# EUROPEAN PATENT SPECIFICATION

(11) **EP 1 254 373 B1**
(45) Date of publication and mention of the grant of the patent: **11.04.2007**
(21) Application number: 01907508.4
(22) Date of filing: 07.02.2001
(51) Int. Cl.: G01N 33/68

(54) **METHOD FOR IDENTIFYING AND/OR CHARACTERIZING A (POLY)PEPTIDE**
VERFAHREN ZUR IDENTIFIZIERUNG UND/ODER CHARAKTERISIERUNG EINES (POLY)PEPTIDES
PROCEDE D'IDENTIFICATION ET/OU DE CARACTERISATION D'UN (POLY)PEPTIDE

(30) Priority: 07.02.2000 EP 00102567
(43) Date of publication of application: 06.11.2002
(73) Proprietor: Max-Planck-Gesellschaft zur Förderung der Wissenschaften e.V., Berlin (DE)
(72) Inventor: CAHILL, Dolores, J., DUBLIN 4 (IE); NORDHOFF, Eckhard, 10829 Berlin (DE); KLOSE, Joachim, Charité, Dept. for Human Genetics, 13353 Berlin (DE); EICKHOFF, Holger, 28857 Syke (DE); SCHMIDT, Frank, 10119 Berlin (DE); LEHRACH, Hans, 14129 Berlin (DE)
(86) International application number: PCT/EP2001/001332
(87) International publication number: WO 2001/057519

(56) References cited:
- NISHIZAWA YUJI ET AL: "Initiating ocular proteomics for cataloging bovine retinal proteins: Microanalytical techniques permit the identification of proteins derived from a novel photoreceptor preparation." EXPERIMENTAL EYE RESEARCH, vol. 69, no. 2, August 1999 (1999-08), pages 195-212, XP002181671 ISSN: 0014-4835
- QUADRONI MANFREDO ET AL: "Proteome mapping, mass spectrometric sequencing and reverse transcription-PCR for characterization of the sulfate starvation-induced response in Pseudomonas aeruginosa PAO1." EUROPEAN JOURNAL OF BIOCHEMISTRY, vol. 266, no. 3, December 1999 (1999-12), pages 986-996, XP002181672 ISSN: 0014-2956
- BUESSOW KONRAD ET AL: "A human cDNA library for high-throughput protein expression screening." GENOMICS, vol. 65, no. 1, 1 April 2000 (2000-04-01), pages 1-8, XP002181673 ISSN: 0888-7543
- CAHILL DOLORES J ET AL: "Bridging genomics and proteomics." PROTEOMICS: FROM PROTEIN SEQUENCE TO FUNCTION., 2001, pages 1-22, XP001036789 BIOS Scientific Publishers Ltd.;Springer-Verlag New York Inc. 9 Newtec Place, Magdalen Road, Oxford, OX4 1RE, UK; 175 Fifth Avenue, New York, NY, 10010-7858, USA ISBN: 0-387-91589-3

## Description

The present invention relates to a method for identifying and/or characterizing a (poly)peptide comprising: (a) analyzing a peptide map of said (poly)peptide, wherein the peptide map comprises at last 1 peptide, and its peptide primary structure fingerprint by mass spectrometry; and (b) comparing data obtained in step (a) with a reference (poly)peptide database, said database comprising mass spectrometric data of peptide maps, wherein a peptide map comprises at least 1 peptide, and peptide primary structure fingerprint data of the peptide(s) of the peptide map of a (poly)peptide or of a variety of (poly)peptides.

With the human genome project well underway and the deadline for completion approaching, the challenges of understanding the function of newly discovered genes have to be addressed. Initial attempts at sequencing the large and complex human genome were intentionally focused on expressed regions, as represented by cDNA repertoires. Estimates of the total gene number vary from 60,000 to over 140,000 (Nature, 401:311 news section 1999)) in the human genome. While the majority of the total number of human genes are now represented as expressed sequence tags (ESTs) in the dbEST database only a tiny minority have yet been assigned a function. For example in the October 22nd 1999 release, the number of entries for human was 1,617,045 (http://www.ncbi.nlm.nih.gov/dbEST/index.html) (Wolfsberg and Landsman, 1997), corresponding to 85,713 clusters in the UniGene set (www.ncbi.nlm.gov/UninGene/Hs.stats.shtml), of which only 9,274 contained known genes. The most straightforward solution to this structure-function discrepancy seems to be the direct correlation between the functional status of a tissue and the expression of certain sets of genes.
However, although the primary amino acid sequences of proteins are encoded by genes, the relationship between genes and proteins is profoundly non-linear. The control and signaling pathways executing the functions of cells are robust and irregular. Cellular activity is transacted through a vast array of signaling, regulatory, and metabolic pathways, each embodied in the functional and structural relationship of many specific molecules. This makes it difficult to predict protein dynamics or structure using genetics. Also, gene-protein dynamics are non-linear as there is no reliable correlation between gene activity and protein abundance (Anderson and Seilhammer, 1997). Structurally, the existence of alternative splice variants of mRNA complicate the relationship between genes and protein. Many proteins undergo post-translational modifications critical to their function but which are not encoded in the protein's corresponding DNA. Furthermore, a protein may be processed in different ways under different conditions, which seems to be of critical importance, for example, in Alzheimer's disease (Masters and Beyreuther, 1998). Another example can be found from experience with the cystic fibrosis transmembrane receptor (CTFR) functions, involved in cystic fibrosis. This disease is caused by a mutation in a single gene, but has a complex pathogenesis, where CTFR functions as a chloride channel but has additional, possible pathological, roles in the regulation of outer membrane conductance pathways. Additionally, the CFTR's expression is highly variable within the lungs, depending on cell type and anatomical location. Such complex functions of a single-gene defect complicate the determination of CFTR in cystic fibrosis and the identification of appropriate cellular targets for therapy (Jiang and Engelhardt, 1998). The overwhelming majority of human diseases are vastly more complex than CFTR, involving large numbers of genes and environmental factors.

Nishizawa (Nishizawa Yuji et al., Initiating ocular proteomics for cataloging bovine retinal proteins: Microanalytical techniques permit the identification of proteins derived from a novel photoreceptor preparation. 'EXPERIMENTAL EYE RESEARCH, vol.69. no. 2, August 1999 (1999-08), pages 195-212) describes the isolation of proteins from photoreceptor cell layers and other compounds of the retina and the subsequent separation by two-dimensional gel electrophoresis and their sequencing.

Quadroni (Quadroni Manfredo et al., Proteome mapping, mass spectrometric sequencing and reverse transcription-PCR for characterization of the sulfate starvation-induced response in Pseudomonas aeruginosa PAO1., EUROPEAN JOURNAL OF BIOCHEMISTRY, vol.266, no. 3, December 1999 (1999-12), pages 986-996) discloses the identification of proteins by the means of two-dimensional gel electrophoresis and subsequently analyzed by N-terminal Edman sequencing and MS sequencing of internal protein fragments. However, both documents refer to theoretical protein data ignoring experimentally determined mass spectrometric data.

Thus, a full understanding of the expression profile of a tissue or organism on the genomic and proteomic levels requires the screening of many samples in parallel, as rapidly as possible.

Accordingly, the technical problem underlying the present invention was to provide a method that allows the identification and/or characterization of proteins in a large scale, short time and in high throughput and low costs.

The solution to this technical problem is achieved by providing the embodiments characterized in the claims.

Accordingly, the present invention relates to a method for identifying and/or characterizing a (poly)peptide comprising:
(a) analyzing a peptide map of said (poly)peptide, wherein the peptide map comprises at least 1 peptide , and its peptide primary structure fingerprint by mass spectrometry; and
(b) comparing data obtained in step (a) with a reference (poly)peptide database, said database comprising mass spectrometric data of peptide maps, comprising at least 1 peptide, and peptide primary structure fingerprint data of the peptide(s) of the peptide map of a (poly)peptide or of a variety of (poly)peptides.

The term "(poly)peptide" as used in accordance with the present invention refers both to peptides and to (poly)peptides, naturally occurring or recombinantly, chemically or by other means produced or modified, which may assume the three dimensional structure of proteins that may be post-translationally processed, optionally in essentially the same way as native proteins. Furthermore, this term encompasses (poly)peptides or proteins having a length of about 50 to several hundreds of amino acids as well as peptides having a length of about 1, 2, 3, 4 and preferably 5 to 50 amino acids. In a further preferred embodiment, said peptide has a length of 6 amino acids. Said (poly)peptide and its map, respectively, in other embodiments comprise 2, 3, 4, 5, 6, up to 10, or more peptides.

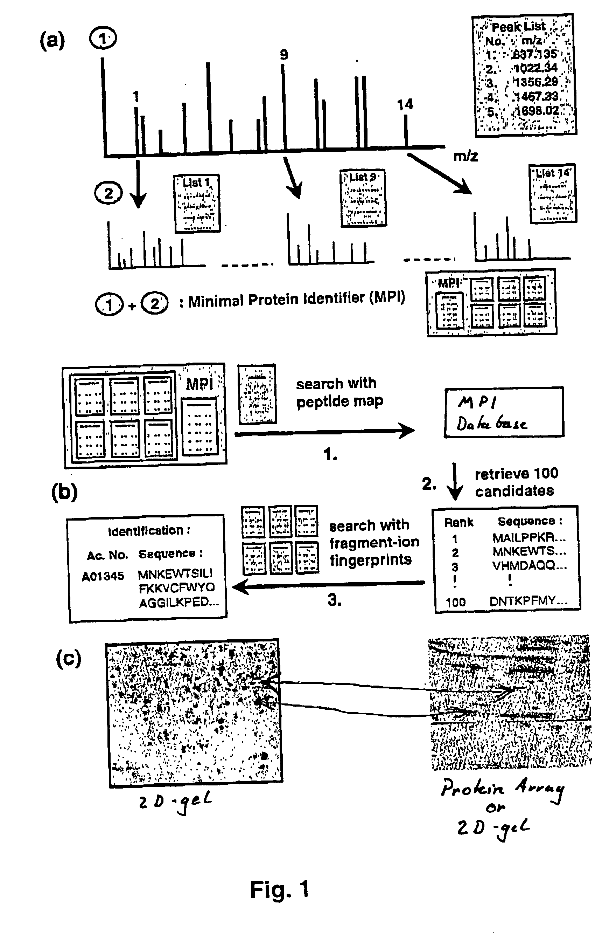

The term "peptide map" as used in accordance with the present invention denotes a set of peptides that is obtained by fragmentation of a given (poly)peptide and, thus, specific for said (poly)peptide. Fragmentation may be effected e.g., by enzymatic digestion of the (poly)peptide, e.g., with trypsin, according to conventional techniques. In specific embodiments, only data from one peptide of a (poly)peptide is contained in said database. In further embodiments, the database comprises data from a variety of peptides wherein each peptide is derived from a different (poly)peptide. It is preferred, however, that said database comprises mass spectrometric data of peptide maps comprising more than one peptide such as 2, 3, 4, 5, 6, 7, 8, 9, 10 or more peptides of a variety of (poly)peptides (see Figure 1).

The term "peptide primary structure fingerprint data"of the peptide(s) of the peptide map" as used in accordance with the present invention denotes the peptide fragmentation pattern as generated by mass spectrometry.

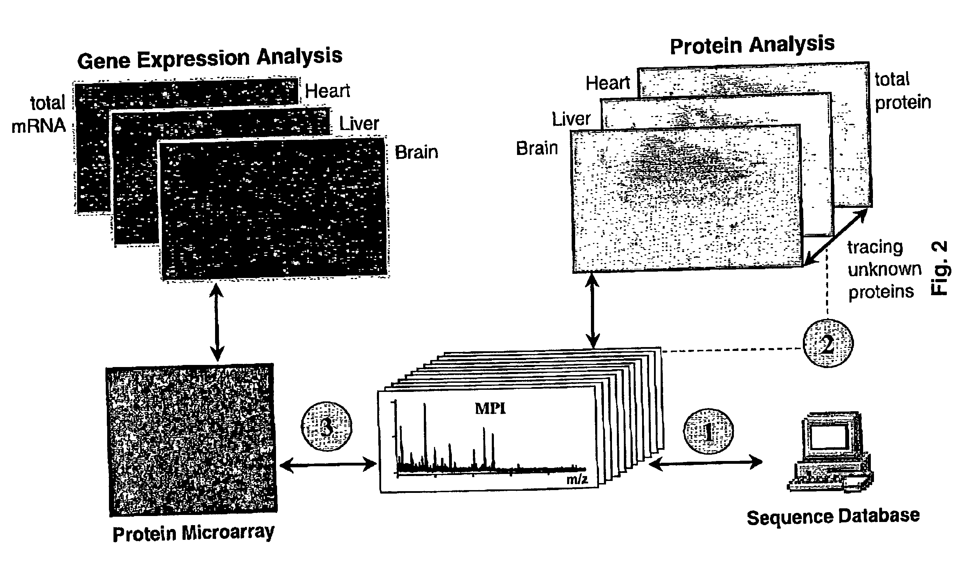

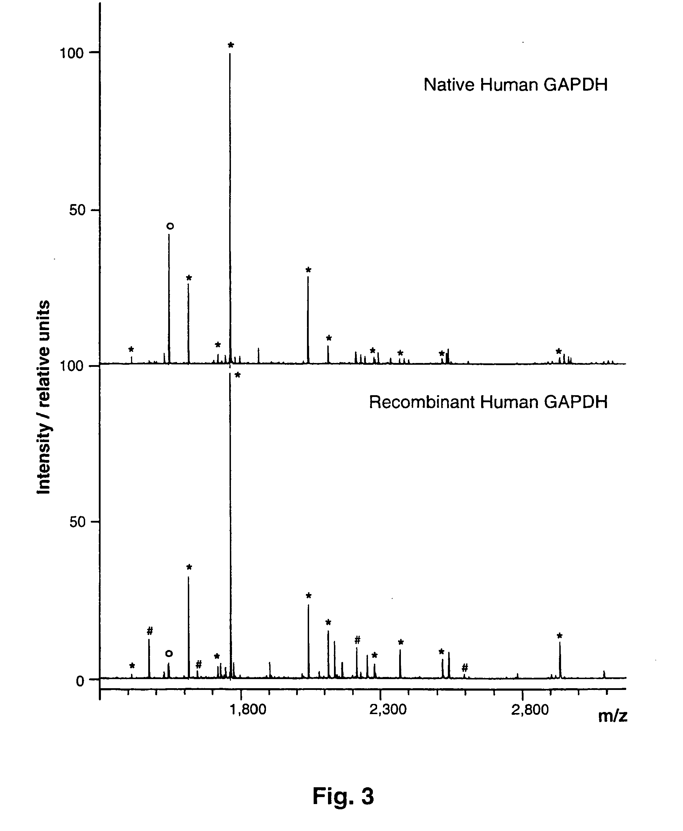

A "variety" of (poly)peptides denotes a number of at least 2 or 3, preferably at least 5 to 50, more preferably at least 50 to 1,000, even more preferred at least 1,000 to 10,000, and most preferred more than 10,000 (poly)peptides.
The method of the present invention advantageously combines data obtained by mass spectrometric analyses of a peptide map, comprising at least 1 peptide, and of its peptide primary structure fingerprint, where "peptide primary structure fingerprint" as used in accordance with the present invention denotes the peptide fragmentation pattern generated by mass spectrometry. Compared to protein identification by mass spectrometric peptide maps, the inclusion of peptide primary structure fingerprints of the peptides of the peptide map strongly improves protein identification in sequence databases and enables unambiguous identification of (poly)peptides (see Figure 2). Peptide primary structure fingerprints may be generated by mass spectrometry-post source decay (MS-PSD) or collision induced decay or laser induced decay well known in the art. This technique is based on a further fragmentation of the peptides and mass spectrometric analysis of the peptide fragments subsequently to the mass spectrometric analysis of the peptides. Preferably, at least 2 to 5 peptide primary structure fingerprints of a (poly)peptide are analyzed by mass spectrometry, more preferred at least 6 to 8, and most preferred at least 10 peptide primary structure fingerprints. Mass spectrometric analysis of peptides is well known in the art and may be performed according to conventional techniques. For example, peptides may be analyzed by matrix-assisted laser desorption ionization mass spectrometry (MALDI-MS) or by electrospray-MS as was performed for human GAPDH from a 2D gel (native human GAPDH) and from GAPDH expressed by E. coli (recombinant human GAPDH) (see Figure 3).
The set of structural information obtained by the method of the present invention for each (poly)peptide, in the following also designated as "minimal protein identifier" (MPI), (see Fig. 1) contains accurate molecular masses of enzymatic cleavage products in conjunction with fragment-ion data. If MPIs of two different (poly)peptides are compared, this advantageously results in a more reliable protein identification since measured MPIs are compared with each other instead of DNA and/or amino acid sequence-predicted structural features (such as identifying spots from 2D gels, as seen in Figure 2).

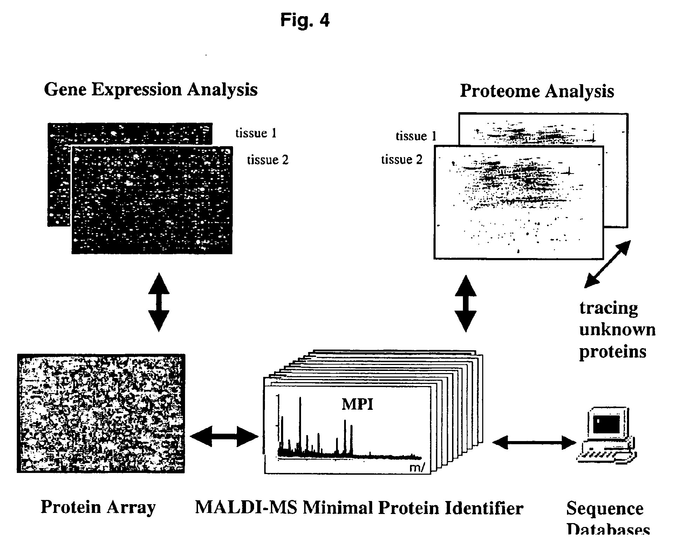

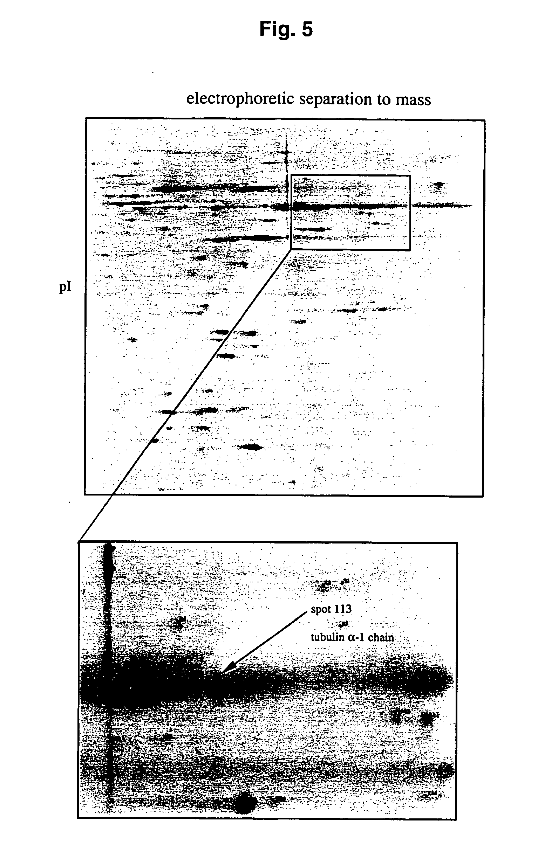

Moreover, MPIs may be electronically stored, thus allowing computer based comparison of different MPIs. This further improves speed and accuracy, reduces costs, and consequently allows high-throughput identification and/or characterization of (poly)peptides (see Figure 4).
A further advantage of the method of the present invention is that it allows identification and/or characterization of a (poly)peptide without knowing its amino acid sequence and/or further structural features (such as identifying spots from 2D gels, as seen in Fig. 5).
It is envisaged in accordance with the present invention that for the identification and/or characterization of a (poly)peptide not necessarily all data obtained in step (a) is compared with the reference (poly)peptide database. Accordingly, for unambiguous identification and/or characterization comparison of the data obtained by the analysis of the peptide map and/or one peptide primary structure fingerprint with the reference (poly)peptide database may be sufficient. Alternatively, comparing the data obtained by analyses of the peptide map and, e.g., in a most preferred embodiment, at least 6-8, preferably 10 or more peptide primary structure fingerprints with the reference (poly)peptide database may result in the finding that no identical mass spectrometric data are present in the reference (poly)peptide database. This would identify the analyzed (poly)peptide as a new entry into the database. Accordingly, such a situation is also encompassed by the term "identifying" as used in accordance with the present invention (see Figure 1).

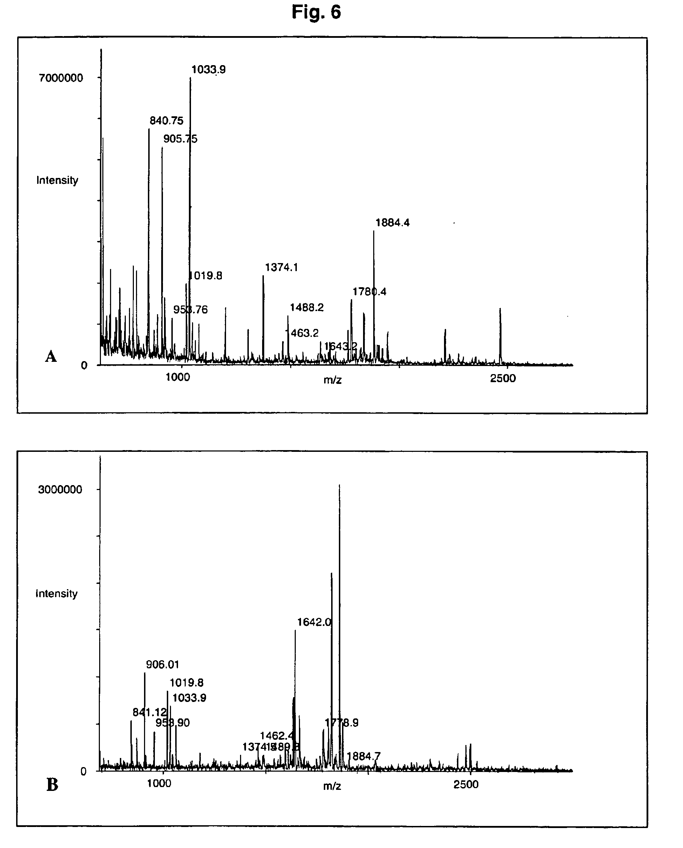

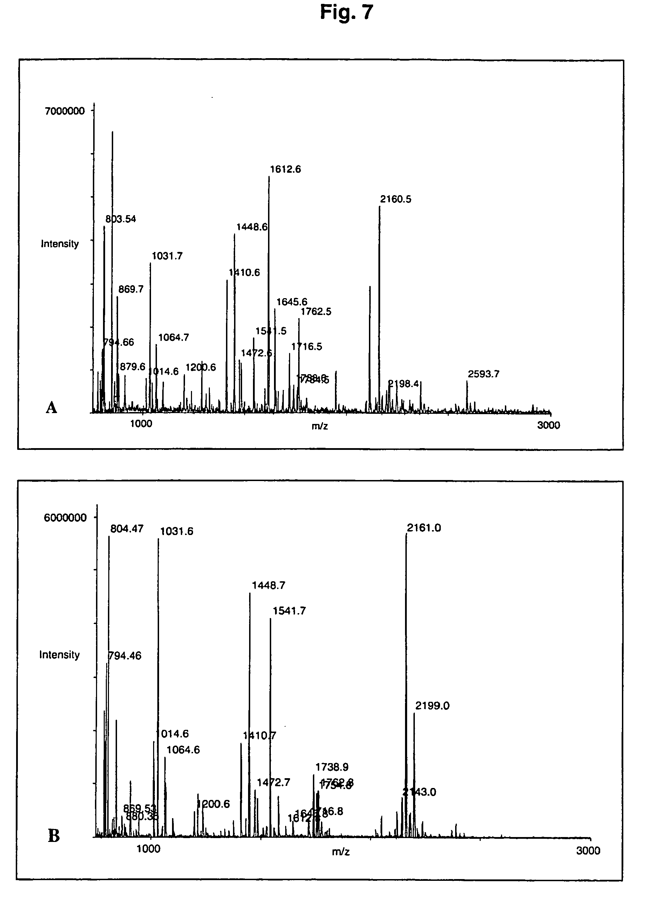

In a preferred embodiment of the present invention, the data obtained in step (a) are recorded as lists of digit numbers corresponding to measured molecular or fragment ion masses or mass/charge (m/z) ratios (see Figures 6 and 7).

In another preferred embodiment, said reference (poly)peptide database in step (b) is produced by the steps of:
(ba) preparing a (poly)peptide sample representative of a species, a tissue, a developmental stage, a specific age, a specific time point a cell, an organelle, a sex, a disease state, a microorganism, a tissue culture cell line, a virus, a bacteriophage, an organism, a plant, an antibody, an antibody library, a protein complex or interacting proteins;
(bb) subjecting said (poly)peptide sample to one- or two-dimensional gel electrophoresis;
(bc) excising (poly)peptides from the gel;
(bd) fragmenting said (poly)peptides;
(be) analyzing the fragments obtained in step (bd) by mass spectrometry; and
(bf) storing the data obtained in step (be) in combination with the source of the corresponding (poly)peptide in a database (for example from a spot in a 2D gel, as in Figure 5, MPI generated as in Figure 1).

Preferably, the above recited organism is an animal, more preferably a mammal and most preferably a human.

The term "specific time point" refers to time points after a tissue, a cell, a non-human organism, including a plant, microorganism etc., an organelle, a tissue culture cell line, a protein complex or interacting proteins, an antibody, an antibody library, a bacteriophage, a virus etc. (of a specific developmental stage, disease stage, sex, age etc.) has been contacted, incubated or treated with a ligand, drug, compound etc., such as described above. Preferably, said tissue etc. is compared to a second sample of said tissue etc. not so contacted or treated.

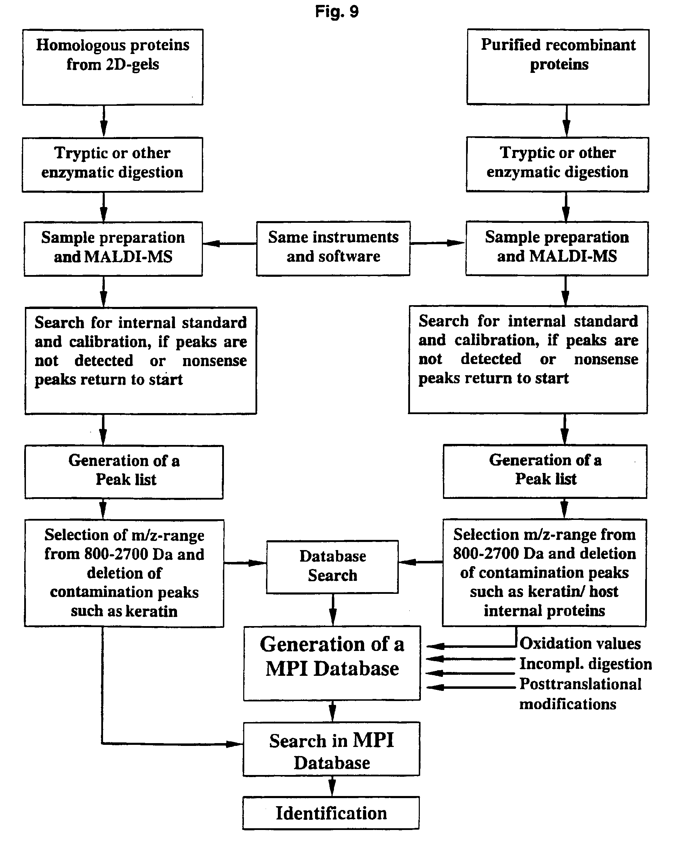

This embodiment of the present invention advantageously not only allows the simultaneous identification and/or characterization of a large number of different (poly)peptides due to the high resolution of the employed two-dimensional gel-electrophoresis (2-DE) but also the assignment of functional parameters to the analyzed (poly)peptide. Accordingly, it is envisaged in accordance with the present invention that 2-DE patterns obtained from, e.g., different species, tissues, developmental stages, cells or organelles, sexes and disease states are compared and subtracted with respect to the presence/absence of protein spots on the different 2-DE patterns, and with respect to different quantitative levels of a (poly)peptide. Evaluation of 2-DE patterns may be performed by laser scanning followed by software assisted spot-recognition and characterization. For presence/absence analysis of protein patterns highly sensitive silver-staining procedures may be used. For quantification purposes, Commassie blue or fluorescent stains, well known in the art, may be employed. This embodiment of the present invention further allows the detection of post-translational modifications, and the person skilled in the art is well aware of, e.g., glycostaining or phosphostaining procedures.
Thus, the method of the present invention allows for the identification and/or characterization of a (poly)peptide if the corresponding MPI matches with a MPI present in the database and, e.g., containing further information with regard to the source of the corresponding (poly)peptide (see Figure 4).
Additionally, due to the MPIs, known as well as unknown individual (poly)peptides may be characterized in a certain population of (poly)peptides and, furthermore, unambiguously identified within and across two or more populations of (poly)peptides (see Figure 4). In other words, once recorded and stored, MPIs enable the tracing of gene products, e.g., in two-dimensional gels run with different biological samples simply by comparing new and previously measured MPIs (see Figure 6). This allows for the provision of further information regarding, e.g., changes of the quantitative levels or of post-translational modifications of the corresponding (poly)peptides that correlate with the expression of said (poly)peptides in, e.g., a certain species, tissue, developmental stage, cell, organelle, sex or disease state.
Another advantage of the method of the present invention is that due to the MPIs a two dimensional (2-D) reference standard pattern can be provided that allows simple and fast comparison of 2-D gels from different laboratories, of different gel formats, independently of the gel resolution and/or applied separation technology, from different patients, tissues, etc. (see above). Once a 2-D reference standard pattern has been established by mass spectrometric analysis of a representative number of spots, preferably of at least 100 spots, more preferred of at least 5,000 spots, most preferred of all discernible spots on the gel, and storage of the corresponding MPIs in a database, in combination with their coordinates of molecular weight and pH in the spot pattern, analysis of only a small number of reference spots (e.g. 20 spots) of, e.g., two gels that are to be compared and allocation to the corresponding spots on the reference standard pattern allows standardization and, thus, comparison of the two gels. This considerably improves the speed of the identification and/or characterization of multiple protein spots by comparison of two different 2-D gels (see Figure 1 and the outline of the procedure (Figure 9)).

The advantages of this method are that the MPI can be used to compare different 2-D gels, as well that the spots, which are differentially present in different 2-D gels (see Figure 1, 2 and 4).

In an additionally preferred embodiment of the of the method of the present invention, said reference (poly)peptide database in step (b) is produced by the steps of:
(ba) preparing a (poly)peptide sample representative of a species, a tissue, a developmental stage, a specific age, a specific time point a cell, an organelle, a sex, a disease state, a microorganism, a tissue culture cell line, a virus, a bacteriophage, an organism, a plant, an antibody, an antibody library, a protein complex or interacting proteins;
(bb) subjecting said (poly)peptide sample to one- or multi-dimensional chromatographic separation steps;
(bc) fragmentation of said separated (poly)peptides;
(bd) analyzing the fragments obtained in step (bc) by mass spectrometry; and
(be) storing the data obtained in step (bd) in combination with the source of the corresponding (poly)peptide in a database.

In a further preferred embodiment of the of the method of the present invention, said reference (poly)peptide database in step (b) is produced by the steps of:
(ba) preparing a cDNA or genomic DNA library representative of a species, a tissue, a developmental stage, a cell, an organelle, a sex, a disease state, a microorganism, a tissue culture cell line, a virus, a bacteriophage, an organism, a plant, an antibody, an antibody library, a protein complex or interacting proteins;
(bb) expressing the cDNA or genomic DNA library obtained in step (ba);
(bc) isolating (poly)peptides obtained in step (bb);
(bd) fragmenting said (poly)peptides;
(be) analyzing the fragments obtained in step (bd) by mass spectrometry; and
(bf) storing the data obtained in step (be) in combination with the source of the corresponding (poly)peptide in a database.

The term "cDNA or genomic library" refers to libraries consisting of complementary DNA or genomic DNA molecules. These cDNA or genomic DNA molecules, referred to throughout this specification, may be full length or non-full length. It is preferred that they are full length. If not full length, said fragments preferably encode a protein domain or an epitope.

This embodiment is particularly useful for applications where it is desired or necessary to have direct access to the genetic information encoding the (poly)peptide the MPI of which has been found in the database. For example, if the MPI of an unknown (poly)peptide is compared with the MPIs of the database, the identification of a MPI in the database matching with the MPI of the (poly)peptide to be analyzed thus does not only provide information with regard to certain functions of the (poly)peptide but also makes immediately available the corresponding genetic information. Thus, only clones of interest need to be sequenced (see Figure 2).
This embodiment also contributes to the speed and convenience of the method of the present invention in another aspect. In the prior art, in order to identify and/or obtain the nucleic acid encoding a (poly)peptide that has been analyzed by mass spectrometry, DNA sequences in the database were computer-translated into amino acid sequences in all possible reading-frames and, e.g., trypsin digestion products of these amino acid sequences computer-generated. The molecular masses of these digestion products were then theoretically calculated and compared with the experimentally obtained mass spectrometric data. Thus, identification of a desired nucleic acid molecule was not only time-consuming and cumbersome but also prone to the identification of false-positive sequences because theoretically and experimentally obtained data were compared to each other. Alternatively or additionally, for the same reason, correct sequences could be missed.

In yet another preferred embodiment of the method of the present invention, said reference (poly)peptide database is generated from (poly)peptides isolated form their natural context.

This advantageously allows for the generation of MPIs inter alia taking into account, e.g., post-translational modifications or specifically processed forms of a (poly)peptide that may not occur when, e.g., a eukaryotic (poly)peptide is recombinantly produced in a prokaryotic host.

However, it is also envisaged in accordance with the present invention that the database also comprises entries comprising structural and functional information of recombinantly produced (poly)peptides, where their corresponding DNA sequences may or may not be known.
The (poly)peptides may be native or denatured.

In a still further preferred embodiment, said (poly)peptide to be identified and/or characterized is a recombinantly produced (poly)peptide.

Methods for the recombinant production of (poly)peptides are well known in the art and include, e.g., production of the (poly)peptide in prokaryotic or eukaryotic hosts. However, the (poly)peptide may also be produced by well known in vitro transcription and translation methods.

In a more preferred embodiment, said recombinantly produced (poly)peptide is comprised in a (poly)peptide library, said library being prepared by expressing a library of nucleic acid molecules comprising a nucleic acid molecule encoding said (poly)peptide.

Vectors that may be used in accordance with the present invention comprise, e.g., plasmids, cosmids, viruses and bacteriophages used conventionally in genetic engineering. Expression vectors derived from viruses such as retroviruses, vaccinia virus, adeno-associated virus, herpes viruses, or bovine papilloma virus, may be used for delivery of the nucleic acid molecule of the invention into targeted cell populations. Methods which are well known to those skilled in the art can be used to construct recombinant viral vectors; see, for example, the techniques described in Sambrook et al., Molecular Cloning A Laboratory Manual, Cold Spring Harbor Laboratory (1989) N.Y. and Ausubel et al., Current Protocols in Molecular Biology, Green Publishing Associates and Wiley Interscience, N.Y. (1989). The vector comprising the nucleic acid molecule of the invention can be transferred into the host cell by well-known methods, which vary depending on the type of cellular host. For example, calcium chloride transfection is commonly utilized for prokaryotic cells, whereas, e.g., calcium phosphate or DEAE-Dextran mediated transfection or electroporation may be used for other cellular hosts; see Sambrook, supra.
Such vectors may comprise further genes such as marker genes which allow for the selection of said vector in a suitable host cell and under suitable conditions.
Expression vectors further comprise expression control sequences allowing expression in prokaryotic or eukaryotic cells. Expression of said nucleic acid molecule comprises transcription of the nucleic acid molecule into a translatable mRNA. Regulatory elements ensuring expression in eukaryotic cells, preferably mammalian cells, are well known to those skilled in the art. They usually comprise regulatory sequences ensuring initiation of transcription and, optionally, a poly-A signal ensuring termination of transcription and stabilization of the transcript, and/or an intron further enhancing expression of said polynucleotide. Additional regulatory elements may include transcriptional as well as translational enhancers, and/or naturally-associated or heterologous promoter regions. Possible regulatory elements permitting expression in prokaryotic host cells comprise, e.g., the PL, lac, trp or tac promoter in E. coli, and examples for regulatory elements permitting expression in eukaryotic host cells are the AOX1 or GAL1 promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the nucleic acid molecule. Furthermore, depending on the expression system used leader sequences capable of directing the polypeptide to a cellular compartment or secreting it into the medium may be added to the coding sequence of the nucleic acid molecule of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL), pCl (Promega), or pQE30 (Qiagen).

In an additionally preferred embodiment of the method of the present invention, said (poly)peptide to be identified and/or characterized is part of a protein complex. Where a protein is isolated and the protein or proteins which form the complex are identical using their MPls. Such complexes can also be run on 1D or 2D gels, and the spots isolated and identified.

In yet another preferred embodiment of the method of the present invention, said (poly)peptide to be identified and/or characterized interacts with another (poly)peptide.
The term "another (poly)peptide" includes antibodies specifically recognizing said (poly)peptide or fragments or derivatives thereof having the same specificity. The term "fragment" of an antibody is well understood in the art (see e.g. Harlow and Lane "Antibodies, A Laboratory Manual", CSH Press, Cold Spring Harbor, USA, 1988) and includes Fab and F(ab')₂ fragments. The term "derivative" is equally well understood and includes scFv fragments. Phage displaying antibodies may also be used, and are well known in the art.

In a further preferred embodiment, said (poly)peptide to be identified and/or characterized is present in a lysate or a whole cell extract. Here (poly)peptides may be isolated which may be difficult to separate on 2D gels, or may be difficult to recombinantly express. Examples of such (poly)peptides may include membrane-bound proteins, trans-membrane proteins, and receptors. As well as proteins which are toxic proteins to the expression host if a recombinant expression system is used.

In a still further preferred embodiment, said mass spectrometric method is MALDI-MS, MALDI-MS/MS, electron spray ionization (ESI), Q-TOF or post-source decay (PSD).

In a particularly preferred embodiment, said library of nucleic acid molecules encode the (poly)peptides as fusion proteins.

In a still further more preferred embodiment said fusion proteins comprise a tag.

Advantageously, tags allow for the convenient isolation, purification, detection and localization for re-arraying purposes of the produced (poly)peptides.

In a most preferred embodiment said tag is a His-tag.

However, other tags like, e.g., c-myc, FLAG, alkaline phosphatase, EpiTag^{™}, V5 tag, T7 tag, Xpress^{™} tag, Strep-tag, a fusion protein, preferably GST, cellulose binding domain, green fluorescent protein, maltose binding protein or lacZ may also be useful in performing the method of the present invention.

In another particularly preferred embodiment of the method of the present invention, expression is inducible.

In yet another more preferred embodiment of the method of the present invention, said nucleic acid molecule is cDNA. This embodiment also includes nucleic acid molecules that constitute a fragment or a full length cDNA molecule.

However, it is also envisaged that said nucleic acid molecule is genomic DNA. This embodiment also includes nucleic acid molecules that constitute a fragment or a full length genomic DNA molecule.

In another preferred embodiment of the method of the present invention, said analysis in step (a) is, in addition to or alternatively to mass spectrometry, effected by surface plasmon resonance, as well known in the art. Such procedures can be performed using BIA core systems, as is well known in the art. This has the advantages of determining interactions, affinity measurements, dissociation and association measurements, as well as identifying and characterising the interacting partners.

In a still further particularly preferred embodiment, prior to expression of said library of nucleic acid molecules, the following steps are carried out:
(aa) amplifying said nucleic acid molecules;
(ab) regularly arraying said amplified nucleic acid molecules; and, optionally,
(ac) hybridizing the regularly arrayed nucleic acid molecules to a variety of oligonucleotides;
(ad) identifying nucleic acid molecules that hybridize to the same set of oligonucleotides; and
(ae) regularly re-arraying per set of oligonucleotides one species of nucleic acid molecules.

It is particularly preferred that the nucleic acid molecules are full length.

In this embodiment arrays, preferably microarrays, are provided comprising an optionally non-redundant set of genomic DNA or cDNA clones (in the following also designated as "UNIgene set" or "UNIclone set") representing a set of mRNAs expressed in a specific species, tissue, developmental stage, cell, organelle, sex, disease state, microorganism, tissue culture cell line, virus, bacteriophage, organism, or plant etc. (see above).
The oligonucleotides may be hybridized sequentially to the array of nucleic acid molecules or as a mixture of oligonucleotides. In the latter case, each species of oligonucleotide is labeled with a specific label. This method also referred to as oligonucleotide fingerprinting is known in the art (Meier-Ewert et al., 1998; Radelof et al., 1998; Poustka et al., 1999; Herwig et al., 1999). Furthermore, the person skilled in the art is well aware of various nucleic acid labels (see, e.g., WO 99/29897 and WO 99/29898).
Regularly arraying said amplified nucleic acid molecules may be effected, e.g., by needle or pin spotting, where liquid containing the nucleic acid molecules will be delivered through adhesion to stainless steel pins. Alternatively, piezo-ink-jet technology may be utilized, where cDNAs, for example, are transferred without touching the surface. Advantageously, a multi-head piezo-jet micro-arraying system is used, which permits the construction of large micro-arrays on a variety of surfaces with a spot density of more than 2000 clones/cm². This methodology is combined with high resolution detection systems, based on laser scanning. As a further alternative to conventional needle spotting, a drop-on-demand technology may be employed. This technology reduces the dimensions of the hybridization arrays by one or two orders of magnitude, the genetic samples are pipetted with a multi-channel micro-dispensing robot, which works on a similar principle to an ink jet printer. Integrated image analysis routines decide whether a suitable drop is generated. If the drop is poorly formed, the nozzle tip is cleaned automatically. A second integrated camera defines positions for automated dispensing, e.g. filling of cavities in silicon wafers. Each head is capable of dispensing single or multiple drops with a volume of 100pl. The dispensers may contain inside a magnetic bead-based purification system. This allows concentration and purification of spotting probes prior to dispensing. The resulting spot size depends on the surface onto which the liquid is dispensed and varies between 100µm and 120µm in diameter. The density of the arrays can be increased to 3,000 spots/cm². The micro-dispensing system has the ability to dispense on-the-fly and takes less than three minutes to dispense 100 x 100 spots, in a square, with 100µm diameter and with 230µm distance between the center of each spots. At this density, it is possible to immobilize a small cDNA library consisting of 14,000 clones, on a microscope-slide surface. This advantageously offers a higher degree of automation since glass-slides are more rigid and easier to handle than membranes.
The array so produced is then hybridized under stringent conditions with a 9-mer oligonucleotides at a temperature between 37 degrees centigrade and 42 degrees centigrade, depending on the GC content, preferably 39 degrees Centigrade, and the positive signals are detected, quantified and stored using image-analysis software. This step is repeated until data from several hybridizations have been collected. By combining all these data an oligofingerprint consisting of the list of probes which hybridize to the nucleic acid molecule may be constructed for each clone. Since the hybridizations are conducted under stringent conditions, these fingerprints are a property of the clones' DNA sequences and, therefore, whenever two clones have similar or identical fingerprints they must have similar or identical sequences and can be clustered together on this basis. Each cluster represents a different gene and has an average, or consensus, fingerprint characteristic of that gene.

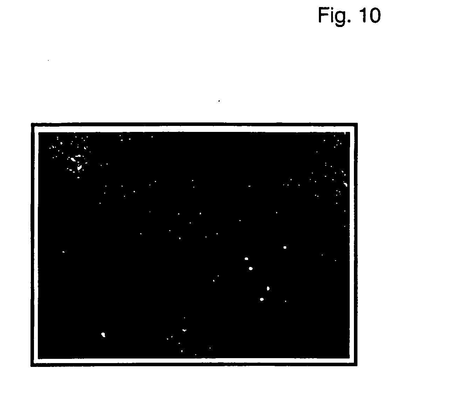

Finally, nucleic acid molecules showing the same sequence may be identified, and a set of non-redundant nucleic acid molecules be regularly re-arrayed by the same procedures described hereinabove.
These arrays will allow the simultaneous measurement of the gene expression level and, therefore, provide an indication of the level of activity, of all genes represented in the array in any sample investigated. When complex mixtures of RNAs or cDNAs or genomic DNA from different, e.g., tissues or developmental stages are hybridized to these DNA chips, this will enable the determination of differences in gene expression profiles.
It is further envisaged that (poly)peptide arrays, in which the positions of the (poly)peptides correspond to the positions of their corresponding cDNA clones on the DNA array, are produced, and the (poly)peptides analyzed as described hereinabove. Protein arrays may be produced by, e.g., automatically spotting proteins from liquid expression cultures using a transfer stamp mounted onto a flatbed spotting robot. If the expression profiles are used to complement the MPIs of the corresponding (poly)peptides, this provides a direct linkage of mRNA and protein populations extracted from, e.g., cells or tissues. (Büssow et al., 1998; also see Figure 10, where a high density protein array of over 2500 proteins are arrayed on a solid surface, and screened with an anti-tubulin antibody. Positive clones were identified to be tubulin).

In a more preferred embodiment, the amplification in step (aa) is effected by PCR.

PCR amplification is a well known technique in the art (see, e.g., Sambrook et al., loc. cit.) and the person skilled in the art knows without further ado how to adapt reaction parameters to certain amplification reactions. Exemplary conditions for 12-mer oligonucleotides, where preferably no mismatch occurs, are at a temperature between 37 degrees centigrade and 42 degrees centigrade, depending on the GC content, preferable 39 degrees Centigrade.

In a more preferred embodiment of the method of the present invention, after expression of said library of nucleic acid molecules, the following steps are carried out in connection with step (b):
(bi) identifying (poly)peptides which, on the basis of the comparative analysis, have a unique minimal protein identifier; and
(bii) re-arranging the clones expressing (poly)peptides identified in step (bi) regularly into an essentially non-redundant set.

With this embodiment, the same advantages are obtained at the protein level as discussed for the preceding embodiment at the nucleic acid level. Namely, a library or collection of essentially non-redundant (poly)peptides is obtained that may then be further analysed. This library, also known as a UNlclone, or a UNlprotein or a UNlgene set, can be used to generate protein arrays, and/or DNA arrays as described in Cahill (2000).

In yet another more preferred embodiment, said regularly arraying and/or said regularly re-arraying is effected on a solid support.

In a still further more preferred embodiment, said solid support is a chip, a glass substrate, a filter, a membrane, a magnetic bead, a silica wafer, metal, a mass spectrometry target or a matrix.
Any of the above solid supports may be coated or uncoated. Coating may be with a gel such as hydrogel or with teflon. Chemical coating is also envisaged. The surface of the solid supports may also be covered by anchor targets.

In a most preferred embodiment of the method of the present invention, said regularly arraying and/or said regularly re-arraying is effected on a porous surface.

The porous surface may be a solid or a non-solid support. Said porous surface may, for example, be a sponge, a membrane, filter, for example PVDF membrane or nylon membrane.

In another most preferred embodiment said regularly arraying and/or said regularly re-arraying is effected on a non-porous surface.

The non-porous surface may also be a solid or non-solid surface/support.

In a further most preferred embodiment of the method of the present invention, said arraying and/or re-arraying is effected by an automated device.
Said automated device, preferably in the form of a robot, may effect spotting, gridding, pipetting or piezo-electric spraying of biological material.

Expression of a library of nucleic acid molecules may be effected, e.g., by the picking of randomly distributed clones from agar plates and arraying these clones into microtitre plates. Advantageously, this is done by picking robots. The colonies are checked by an image analysis system to address the position for picking. The software, furthermore, identifies clone positions and translates the position into robot movement. The next step is the profiling of protein products encoded by differentially expressed genomic DNA or cDNA clones, including the simultaneous expression of large numbers of cDNA clones in an appropriate vector system and high-speed arraying of protein products. For example, using robotic technology, a human fetal brain cDNA expression library may be arrayed in microtitre plates, and bacterial colonies may be gridded onto PVDF filters. *In situ* expression of recombinant fusion proteins may be induced and detected using an antibody against a 6xHis-tag-containing epitope. Using such an approach, the genes in these libraries can be studied on the DNA and protein levels simultaneously, and provide sources of recombinant genes and proteins to make DNA and protein chips. This approach may also achieve the large-scale systematic provision of recombinant proteins for functional studies by making and arraying cDNA expression libraries and by allowing the direct connection from DNA sequence information on individual clones to protein products and back again on a whole genome level. This makes translated gene products directly amenable to high-throughput experimentation and generates a direct link between protein expression and DNA sequence data (Cahill et al., 2000).

In another more preferred embodiment of the method of the present invention, said variety of oligonucleotides comprises at least 2, preferably at least 10, and most preferred at least 150 different oligonucleotides.

In another preferred embodiment of the method of the present invention prior to step (aa), the following steps are carried out:
(aa') optionally reverse transcribing mRNA from a species, a tissue, a developmental stage, a cell, an organelle, a sex, a disease state, a microorganism, a tissue culture cell line, a virus, a bacteriophage, an organism, or a plant into cDNA;
(aa") cloning cDNA, optionally obtained in step (aa'), or genomic DNA into an expression vector.

Isolation of mRNA and reverse transcription into cDNA are well known methods in the art (see, e.g., Sambrook loc. cit.). Accordingly, RNA may be prepared, and mRNA isolated via, e.g., oligo-dT cellulose. Subsequently, e.g., oligo-dT primer may be hybridized to the poly-A tails of the mRNA, and mRNA reverse transcribed via, e.g., AMV reverse transcriptase. After second strand synthesis the so obtained cDNA may then be cloned into an expression vector using well known techniques. Suitable expression vectors have been described herein above.
If extracted mRNA populations are, via reverse transcription and cloning, expressed as recombinant fusion proteins, their encoded MPIs can easily be determined by mass spectrometry (see Figure 4 and also Figure 3B, Figure 6B Figure 7). By comparing the MPIs recorded from native proteins, isolated by 2-DE, with their recombinant pedants, corresponding transcription and translation products are identified. In that way, a large number of biologically active gene products are envisaged to be characterized and linked to their genes without knowing their sequence (see Figure3, Figure 4 and Figure 5).

In a still further preferred embodiment, the following further steps are carried out:
(ai) after expression of said (poly)peptide, isolating the expressed fusion proteins by means of the tag;
(aii) fragmenting the fusion proteins;
(aiii) analyzing the fragments obtained in step (aii) by mass spectrometry; and
(aiv) storing the data obtained in step (aiii) in a database.

In this embodiment, clones may be grown, e.g., in microtitre plates, protein expression induced, and the produced fusion proteins purified via their tag and, e.g., magnetic beads. Furthermore, it is envisaged that the bound fusion proteins are digested "on-particle" by, e.g., trypsin, and the emerging peptides subjected to MALDI-MS and MS-PSD. As a result, an MPI profile is generated for each (poly)peptide produced by the optionally non-redundant clones that unambiguously specifies each entry, and allows its rapid identification (see Figure 6).

In a more preferred embodiment, said isolation is effected by metal chelate affinity purification.

In a most preferred embodiment, said metal chelate affinity purification employs Ni²⁺-NTA ligands immobilized onto magnetic particles. Alternatively, they may be immobilized on agarose; see Fig. 3.

However, Ni²⁺-NTA ligands may also be immobilized onto Ni²⁺-NTA agarose or a matrix of a column.
This embodiment of the purification is most preferred because the yield and the purity of the product is high, the method is cheap and fast, and appropriate for automation and high-throughput handling of large numbers of proteins.

Another most preferred embodiment of the method of the present invention further comprises:
(af) hybridizing genomic DNA, PNA, cDNA or RNA molecules to the optionally re-arrayed nucleic acid molecules of step (ae); and
(ag) identifying genomic DNA, PNA, cDNA or RNA molecules that hybridize to the optionally re-arrayed nucleic acid molecules on the array.

Any of the above recited hybridizing molecules may be in the form of synthetic oligonucleotides. Yet, other origins such as naturally derived or recombinantly produced are also envisaged.

This embodiment of the present invention advantageously provides the link of genes to their expression products and vice versa (see Figure 2 and Figure 4).

In a more preferred embodiment of the method of the present invention, expression is effected in procaryotes.

In an even more preferred embodiment said procaryotes are bacteria.

In a most preferred embodiment said bacteria are E. coli (see Figure 6B and Figure 7B).

In a more preferred embodiment of the method of the present invention, expression is effected in non-human eukaryotes or eukaryotic cells.

In an even more preferred embodiment said non-human eukaryotes are yeast, such as S. cerevisiae.

In a most preferred embodiment said yeast belong to the species Pichia pastoris (see Figure 7A).

In another more preferred embodiment said eukaryotic cells are mammalian or insect cells.

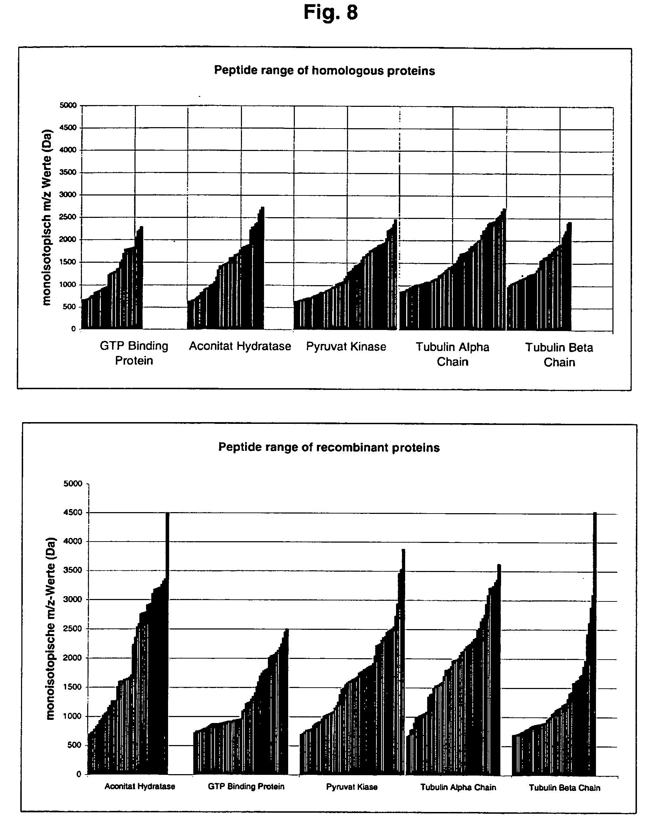

In a preferred embodiment of the method of the present invention, said peptides have a molecular weight in the range of 600 to 4500 Daltons. This range of peptides has specific advantages, in particular, if the peptides to be analysed are of heterologous nature as compared to the peptides stored in the data base, as is evident from the appended example (see Figure 8: Peptide range of recombinant proteins).

The distribution of m/z values is important for the determination of MPls. The MPIs were calculated for the number of peaks in a spectrum within the range 800 Da to 2000 Da. This range was selected because the minimal and maximal region of detection is on average 600-2750 Da for the homologous and 600-4500 Da for the heterologous protein, respectively (Figure 8: Peptide range for homologous proteins). Comparing both spectra systematically, specific peptides dropped out. Therefore, the threshold range mentioned above was selected for calculating the MPI, which also results in a smaller data set, increasing the search speed.

In a most preferred embodiment, said peptides have a molecular weight of 600 to 2750 Daltons. This embodiment is particularly advantageous if the peptides are of homologous nature.

In a preferred embodiment of the method of the present invention, said comparing in step (b) comprises normalization for chemical or post-translational modifications. Normalization can be effected e.g. on the basis of the teachings of the appended example.

In a most preferred embodiment, said chemical modification is oxidation.

Post-translational modifications include glycosylation and phosphorylation, acetylation, sulfation and myristoylation.

As described hereinabove, by the method of the present invention (poly)peptides may be identified and/or characterized. In other words, the method of the present invention allows for the provision of structural and functional features of (poly)peptides independently of whether they are known or unknown.
As also described hereinabove, the method of the present invention further allows for the combination of these biological and biochemical parameters of different (poly)peptides with their gene expression profiles (see Figure 2 and Figure 4).
Finally, if genomic DNA molecules are hybridized to the arrays of nucleic acid molecules produced in accordance with the present invention, the here described method not only allows for the functional and structural identification and/or characterization of (poly)peptides but also for the identification and isolation of the genes encoding these (poly)peptides, thus, further contributing to the elucidation of the genome-proteone interrelation, e.g., in a particular cell or tissue, under normal conditions, disease conditions and activated (for example drug-treated) conditions.

The method of the present invention may also be useful for the development of pharmaceuticals and/or diagnostics. Accordingly, the method of the present invention may be focused on the identification and/or characterization of (poly)peptides that show, e.g., altered expression levels and/or structural modifications like, e.g., post-translational modifications or amino acid substitutions, additions and/or deletions in different disease states or if normal conditions and disease conditions are compared. This may, in turn, lead to the identification of corresponding defects on the DNA level, valuable information for pharmaceutical and/or diagnostic purposes, and/or the identification of compounds counteracting the abnormal expression levels and/or structural modifications and, thus, being potential drug candidates.

The figures show:
- **Figure 1:**: **(a)** Acquisition of minimal protein identifiers (MPIs) by MALDI-MS. The proteins are digested with a specific protease, e.g. trypsin, and the cleavage products' molecular masses are determined. Subsequently, for each protein fragment-ion spectra of a selection of prominent cleavage peptides are recorded. The peptide mass map extracted from the first spectrum provides a fingerprint of the protein's primary structure whereas the fragment-ion peak lists yield fingerprints of the cleavage peptides' amino acid sequences. These data are combined and stored as MPIs, one for each protein.
**(b)** Strategy for identifying proteins in sequence databases. Searching the database for a specific peptide mass map retrieves a list of candidate protein sequences (e.g., 100 sequences). This list is searched for cleavage peptides that match the recorded fragment-ion fingerprints and ranked accordingly. The advantage of the proposed sequential strategy is high search specificity and short search times since the second selection round is applied only to small subset of the whole database.
**(c)** Strategy for comparing 2-DE protein gels. For assigning protein spots, instead of their patterns their recorded MPIs are compared *in silico* (i.e. by computer-based methods). This assignment is independent of the used gel formats, the applied separation technique and followed 2-DE protocol. Correlation of 2-DE protein spot patterns and ordered protein micro arrays. For all recombinant proteins spotted onto the array, MPls were recorded before and stored in a database. Native proteins separated by 2-DE can now be assigned to their recombinant derivatives by comparing their determined MPIs with the above database entries.
- **Figure 2:**: The proposed concept The Bridge. Native proteins are correlated to their genes and RNA expression levels by the use of minimal protein identifier (MPIs, see Figure 1) determined by mass spectrometry. A Unigene-Uniprotein set (also known as a UNlclone set) extracted from cDNA libraries provides both, unique gene representatives via PCR readily accessible for gene expression analysis on cDNA microarrays, and the corresponding expression products as (His)₆-fusion proteins ready for affinity purification. The purified proteins are proteolyzed and analyzed by MALDI. Native protein populations extracted from cell cultures or tissue are separated and characterized by 2-D electrophoresis followed by in situ proteolysis and MALDI-MS. The collected MPIs are compared with the MPIs obtained from the recombinant protein library, and vice-versa. Thereby, thousands of biologically active gene products are linked to their genes. This linkage is independent of any sequence information.
- **Figure 3**:: MALDI-TOF-MS tryptic peptide maps of native and recombinant human GAPDH. Native GAPDH was isolated from total human fetal brain protein extract by large-format 2-D electrophoresis and digested in situ. The spectrum (top panel) was obtained from a 5-µl aliquot of purified overnight digestion supernatant. Recombinant human GAPDH equipped with an RGSHis₆-tag at the N-terminus was expressed in *E. coli.* Tagged proteins were metal-chelate affinity purified from crude cell extract using NTA-ligands immobilized on agarose (Qiagen, Germany) under denaturing conditions. The purified proteins were digested in situ. The spectrum (bottom panel) was obtained from 0.5µl of a total of 150 µl digestion supernatant. Marked signals: * Tryptic cleavage peptides detected in the digestion supernatant of native GAPDH according to the NCBI database (accession number: 12,0649, release 05.05.1999). All these peptides were also detected in the digestion supernatant of recombinant GAPDH. # Additional tryptic cleavage peptides detected in the digestion supernatant of recombinant GAPDH. Peptide detected in both digestion supernatants that could not be assigned to GAPDH and not to any trypsin autolysis product.
- **Figure 4**:: The novel concept, "The Bridge". Homologous proteins from 2D gels are correlated to their genes and RNA expression levels by the use of minimal protein identifiers (MPIs), as determined by mass spectrometry. A UNIgene-UNIprotein set (also known as a UNIclone set) can be derived from cDNA expression libraries provides both genes and proteins, sequence information for each clone in the set can also be obtained. The UNlgene set can obtained by PCR of all clones, which can be used for gene expression analysis on cDNA microarrays [Eickhoff, 2000]. The corresponding proteins can be used to generate a UNIprotein array or, following proteolysis, can be analysed by MALDI-MS to generate specific MPIs for each protein followed by storage in a MPI-database. By comparing these MPIs, to MPIs generated from homologous proteins extracted from tissues and separated on 2D gels, a characterisation and identification of 2D gel separated proteins is possible.
- **Figure 5:**: 2D-gel containing electrophoretically separated proteins from human foetal brain tissue. The proteins were separated in the first dimension by their isoelectric point (pl), followed by separation in the second dimension separation based on their molecular weight. The arrows in the enlarged section indicate identified spots of tubulin α 1 chain and its isoforms.
- **Figure 6:**: Comparison of the spectra of homologous and recombinant pyruvate kinase. **A:** Spectrum of the homologous pyruvate kinase, following extraction from 2D gels and tryptic digestion. **B:** spectrum of the recombinantly expressed pyruvate kinase, also following purification and tryptic digestion. The identical peaks from both the homologous and the recombinant protein, are indicated by their size.
- **Figure 7:**: Comparison of the spectra of recombinant human GAPDH, expressed in two different expression hosts. **A**: Spectrum of GAPDH expressed in *P. pastoris.* **B:** Spectrum of GAPDH expressed in *E. coli.*
- **Figure 8:**: The distribution of m/z values of the homologous proteins and the recombinant expressed proteins analysed.
- **Figure 9:**: Flow sheet demonstrating processes for identifying proteins by using the technology of the present invention.
- **Figure 10:**: A high density protein array, with more than 2500 essentially non-redundant proteins arrayed on a solid support. By screening protein chips containing approximately 2500 different proteins from the UNIprotein set spotted on PVDF membrane with anti-tubulin (human) antibody, α-tubulin clones were identified. The expressed proteins from these clones may also be used for the generation of MPIs.

The example illustrates the invention.

### Example : Identification of proteins, using 2D gel electrophoresis and MPI from a selection of recombinantly produced proteins (see Figure 3, Figure 6 and Tables 1 and 2)

### Material and Methods

**Strains, transformation and media.** *Escherichia coli* strains XL-1Blue, BL21 (D3)pLysS (Invitrogen) and SCS1 (Stratagene) were used for cloning and expression as described [Büssow et al., 1998, Lueking et al., 2000].
*Pichia pastoris:* strain GS115 (his4, Mut+; Invitrogen) was used for eukaryotic protein expression as described [Lueking et al., 2000].

**Protein expression and purification**. The bacterial protein expression in strain SCS1 were performed as described in [Büssow et al., 1998], and the expression in strain BL21(D3)pLysS as described in [Lueking et al., 2000]. The proteins were purified as previously described [Büssow et al., 2000].

### Mass spectrometry

### Tryptic digestion of 2-D gel separated proteins from human brain

Coomassie G250-stained large-format 2D gels of human brain total protein extract were prepared, according to the protocol of Klose (1975), Humangenetik 26, 231-243 where cylindrical gel samples of 1 mm diameter were excised and then destained by incubation with 400 µL 25% isopropanol for 30 min. The destained gel samples were dried in a vacuum centrifuge for 10 min, followed by addition of 5 µL digestion buffer (5 mM DTT, 5 mM n-octylglucopyranoside (n-OGP), 20 mM Tris, pH 7.8) containing 12 ng/µL modified porcine trypsin (sequencing grade, Promega). Following overnight incubation at 37°C, 5 µL 0.4% TFA, 5 mM n-OGP were added and incubated for 1 h, at room temperature. Samples were stored at -20°C prior to MALDI-MS sample preparation.

### Tryptic digestion of heterologous expressed proteins

The proteins were electrophoretically separated by SDS-PAGE (12.5% polyacrylamide, bisacrylamide 30:0.8). The gels were stained with Commassie Blue and destained and protein spots were visualised. The spots were excised from the 2-D gels and the proteins were extracted and tryptically digested as described above, as well known in the art.

### MALDI Sample Preparation

Sample desalting and enrichment was achieved using micro-scale reversed-phase purification tips (ZipTip-C₁₈, Millipore), following the protocol provided by the manufacturer.

### CHCA surface affinity preparation

Samples were prepared on pre-structured MALDI sample supports (Schuerenberg et al., 2000), using alpha-cyano-4-hydroxycinammic acid (CHCA) as the matrix, according to a recently described protocol (Gobom et al., 2001).

### MALDI-TOF-MS

Mass spectra of positively charged ions were recorded on a Bruker Scout 384 Reflex III instrument (Bruker Daltonik, Bremen, Germany) operated in the reflector mode. 100 single-shot spectra were accumulated from each sample. The total acceleration voltage was 25 kV. The XMASS 5.0 and MS Biotools software packages provided by the manufacturer were used for data processing. For the calibration of the tryptic digested protein samples, known auto-proteolytic products of trypsin were used for internal calibration.

### Database searching

For protein identification, human protein sequences in the SwissProt database (www.expasy.ch/) and PROWL (Rockefeller University) databases (www.prowl.rockefeller.edu/), were searched using the Mascot Software (Matrix Science Ltd., U.K.) The probability score calculated by the software was used as the criterion for correct identification. A further criterion was applied, namely, that a minimum of three peptides were required to match the highest ranking sequence entry, compared to the next unrelated candidate. A mass deviation of 30 ppm was tolerated in the searches, and for proteins isolated from 2-DE, oxidation of methionine residues was considered a possible modification.

### Generation of MPI

For the generation of MPIs, all possible m/z-values in the databases searched were transformed using the software "m/z-freeware edition" (Proteometrics, LLC) (www.canada.proteometrics.com/). The theoretical enzymatic cleavage of the database proteins was performed using the GPMAW software version 3.15 (Lighthouse data) (www.welcome.to/gpmaw).

### Results

### Comparison of MALDI-TOF-MS of recombinant proteins and their corresponding native proteins from 2D gels.

For comparison by mass spectrometry, 5 proteins (Aconitate hydrogenase, pyruvate kinase, GTP binding protein, tubulin α-1 chain and tubulin β-3 chain) that were previously identified and analysed on 2-DE gels (Figures 3, 6) by MS were selected from the (oligofingerprinted) Unigene/UNlprotein set [Cahill et al., 2000] and expressed in *E. coli.* The recombinant proteins were expressed, purified and analysed by MS.
The spectra of the recombinantly expressed proteins and the homologous proteins from 2-DE gels (as is shown for Figure 3 (human GAPDH) and Figure 6 (human pyruvate kinase)) were compared.
To determine the feasibility of this approach, the coverage and the MPI value were calculated, both in percent. The coverage, as a percentage, was determined on comparing the number of actually identified peaks with the number of all theoretically possible peaks, after *in silico* digestion. The MPI value is the number of identical peaks, from the homologous and heterologous protein, based on the total number of peaks obtained from the heterologous protein, as a percentage.

In Figures 6A (native, homologous 2-D gel) and 6B (recombinantly expressed, heterologous), the peaks are marked by their size which are present in the spectra from the recombinant proteins (e.g. pyruvate kinase) and from the native proteins from the 2-D. Both spectra were obtained using the PROWL database. The database hits and the peaks, which were present in both, the recombinant and 2-D gel proteins are shown in Table 1. 11 peaks were obtained from the recombinant pyruvate kinase protein, which corresponded with the peaks obtained from the homologous form of pyruvate kinase (MPI). 10 peaks were obtained from the recombinant protein, all 10 of which were found in the 54 theoretically possible peaks obtained for pyruvate kinase in the PROWL database (Table 1). Therefore, the coverage obtained was 18.5%. For the homologous pyruvate kinase protein, 12 of the possible 54 hits were found, resulting in a coverage of 22.5 % as shown in Table 2. The MPI value of pyruvate kinase was 42.0%. The average coverage of the recombinant proteins was found to be 26.6%, and the average coverage of the homologous proteins was 31.9% (Table 2). The average MPI-value of all 5 proteins was found to be 30.62%. Based on these results; it is suggested that an MPI value of approximately 30% may be sufficient to identify proteins from 2 D gels or other sources.

**Table 1: Monoisotopic molecular masses of peptide ions detected in the peptide maps of the recombinant and native pyruvate kinase (shown in Figure 6) that match the calculated masses for the protein**

| **m/z recombinant pyruvate kinase** | **m/z homologous pyruvate kinase** | **m/z identical peaks** | **m/z theoretical identical peaks of the 54 possible peaks** |
|---|---|---|---|
| - | 787.64 | - | 787.42 |
| 841.12 | 840.75 | + | 840.53 |
| 869.04 | - | - | 868.49 |
| 906.01 | 905.75 | + | - |
| 953.90 | 953.76 | + | 953.48 |
| 1019.83 | 1019.80 | + | 1019.52 |
| 1033.90 | 1033.90 | + | - |
| - | 1198.00 | - | 1197.65 |
| - | 1303.00 | - | 1302.68 |
| 1374.46 | 1374.10 | + | - |
| 1462.35 | 1463.20 | + | 1462.82 |
| 1489.31 | 1488.20 | + | - |
| - | 1637.22 | - | 1636.89 |
| 1641.99 | 1643.20 | + | 1642.77 |
| 1664.99 | - | - | 1665.83 |
| - | 1765.50 | - | 1764.99 |
| 1778.86 | 1780.40 | + | 1779.88 |
| 1858.71 | - | - | 1859.91 |
| 1884.70 | 1884.40 | + | 1883.90 |

**Table 2: Number of matched peptide masses of recombinant and native proteins to the theoretical digestion (complete digest). Additionally, the number of matched masses of native and recombinant proteins are shown.**

| **protein** | **database hits-homologous protein** | **database hits-recombinant protein** | **coverage homologous protein** | **coverage recombinant protein** | **identical m/z-values** | **identical m/z-value/total detected peaks of recombinant protein (MPI-value)** |
|---|---|---|---|---|---|---|
| pyruvate kinase | 12 | 10 | 22.2% | 18.5% | 11 | 42.4% |
| GTP binding protein | 8 | 8 | 36.4% | 36.4% | 17 | 60.7% |
| aconitate hydratase | 13 | 10 | 31.0% | 23.8% | 5 | 18.5% |
| tubulin α-1 chain | 11 | 5 | 35.5% | 16.3% | 4 | 23.5% |
| tubulin β-3 chain | 10 | 11 | 34.4% | 37.9% | 2 | 8.0% |
| average value | 10.8 | 8.8 | 31.9 % | 26..6 % | 7.8 | 30..62% |

### The effect of oxidation of homologous proteins from 2 DE gels and their consequence on the MPIs.

Due to the long staining times of 2D gels with Coomassie® G250, homologous proteins may be oxidised, particularly methionine. As generally, the recombinantly expressed proteins are more concentrated and, therefore, require only short staining times, these proteins are less oxidised. As a consequence a peptide containing an oxidised amino acid would have an increased mass, for example, when methionine is oxidised, an increase of 16.00 m/z units is obtained in the monoisotopic state. This corresponds to the addition of one oxygen molecule. For example, each of the peptides 6, 19 and 35 from tryptically digested tubulin β-3 chain contain one methionine. Comparing the spectrum of the homologous protein with those of the recombinantly expressed tubulin β-3 chain, the peaks 6, 19, 35 of the homologous protein show a precise increase of 16 Da (see Table 3). This difference of 16 Da may be result in some difficulties in the identification of unknown proteins from 2D gels when compared to a database based on spectra of heterologous expressed proteins. Modifying the MPI-database by addition of such values of oxidised peptides, will improve the number of identical peaks obtained, as well as improving the probability of correct identification. For tubulin β-3 chain, such a database modification will lead to the ability to increase the number of peaks used to determine the MPI value from 2 to 5 peaks, resulting in a more reliable MPI value.

**Table 3: Tryptic peptides from native tubulin-β-3 chain, detected at m/z values corresponding to oxidation of one methionine residue (+16 Da)**

| **peptide number** | **amino acid number** | **amino acid sequence** | **theoretical mass [Da]** | **theoretical mass following oxidation of methionine [Da]** | **measured mass following oxidation of methionine [Da]** |
|---|---|---|---|---|---|
| 6 | 63-77 | Ala-Ile-Leu-Val-Asp-Leu-Glu-Pro- Gly-Thr-Met-Asp-Ser-Val-Arg | 1614.83 | 1630.82 | 1630.80 |
| 19 | 253-262 | Leu-Ala-Val-Asn-Met-Val-Pro-Phe- Pro-Arg | 1142.63 | 1158.63 | 1158.61 |
| 35 | 381-390 | Ile-Ser-Glu-Gln-Phe-Thr-Ala-Met- Phe-Arg | 1228.59 | 1244.59 | 1244.60 |

### The distribution of m/z values.

The distribution of m/z values is important for the determination of MPIs. In general, the value of MPIs (%) was calculated for the number of peaks in a spectrum within the range 800 Da to 2000 Da. This range was selected because the minimal and maximal region of detection is on average 600-2750 Da (see Figure 8: top panel) for the homologous and 600-4500 Da for the heterologous protein (see Figure 8: bottom panel), respectively. Comparing both spectra systematically, specific peptides dropped out. Therefore, the threshold value mentioned above was selected for calculating the MPI value, which also results in a smaller data set, preventing reduced search speed due to large amounts of data stored in the database (see Figure 1 and the overview, Figure 9).

### Influence of expression by different hosts on the MPIs

The generation of a database containing MPIs may use heterologous expressed proteins from different hosts. Therefore, it is important to analyse whether the expression by different hosts influences the peptide spectrum. Since cDNA expression libraries are mainly generated in *E. coli* (Büssow, 1998) and, only recently, in yeast expression libraries, as described (Lueking, 2000). Here, *E. coli* and the yeast *Pichia pastoris* were used as reference expression hosts. Human GAPDH were expressed in both hosts using the dual expression vector (Lueking et al., 2000) suitable for expression in *P. pastoris* (see Figure 7A) and *E. coli* (see Figure 7B). 22 identical peaks were found from a total number of 50 peaks from GAPDH when expressed in *E. coli,* and 56 peaks when expressed in *P. pastoris.* Comparing these to the 33 theoretically obtained peaks, 12 and 14 peaks respectively, were found to be identical, which correspond to 36 % and 42 % coverage. This indicates that MPIs can be determined regardless of the expression host, offering the possibility to use different expression systems and libraries.

These data provide a proof of principal of the method of the present invention to improve the identification of proteins, e.g. from 2 D gels, using generated MPI from proteins such as recombinantly expressed proteins. The above data qualify the present invention for a high throughout and, potentially fully automated method to identify proteins using mass spectrometry.

With the prior art methods, it was only possible to obtain about 50% coverage when identifying proteins by MALDI-MS. There are a number of reasons for this, namely, due to the redundancy in the genetic code, the incorrect amino acid sequence is generated. Other reasons may include that the protein is absent in the databases searched, or sequencing errors and contaminating sequences in the databases.

Therefore, a technique is described to improve this by generating mass spectrometry fingerprints of proteins, such as recombinant proteins. It was also shown that it is possible to carry out a high throughput and reliable method to identify proteins by mass spectrometry. The method of the invention also enables high throughput or automatic generation of MPI, which includes the standardisation of sample preparation procedures (for a general outline of the procedure, see Figures 1, 2, 4 and 9).

However, for the establishing of such an MPI database, the following points should be noted. For the identification of a known, or previously unknown protein, it was determined that an MPI value of at least 15% is sufficient, which may correspond to about 5 peaks that match to the peaks obtained from the homologous protein. Based on the results shown in Figure 8, it was determined that these selected peptides should be in the size range of 800 Da to a maximum of 4500, more preferably 2750, most preferably 2000 Da. If the peaks are smaller than 800 Da, they are mostly due to individual amino acids and smaller peptides and will not be used for the MPI generation. Additionally, as can be seen in Figure 8, peptides obtained from recombinant proteins were in the higher m/z range, when compared to the same proteins from 2-D gels. It is suggested that such peaks result from incomplete trypsin-digestion due to high protein concentration of the recombinant proteins. Therefore, peaks in the m/z region over 2750 Da, more preferably over 2000 Da, should be excluded in the generation of MPI stored in this database.

Preferably, the relative intensity units are correctly selected, so that only the well-defined peaks above background are selected. It is also preferred that an internal standard is measured, such as the auto-digestion peaks of trypsin, which will be used for the automatic calibration of the software, and also to determine if the spectrum is worth measuring.

The MPI database will also include information such as the expected peptide mass changes resulting from modifications of proteins such as oxidation, incomplete digestion of trypsin, and that these known variability factors as that methionine when present in a peptide, it is not always completely oxidised. Including such information in the MPI-database facilitates the improved identification of the various peptides obtained.

As can be seen from Table 1, peptides were obtained that were not present in the theoretical peak list. However, this did not hinder the generation of useful MPIs.
These additional peaks may be explained by the presence of premature terminated proteins, which may have resulted from differences in codon usage when the protein is expressed in different host expression systems. Other possibilities may be due to the degradation of the proteins during storage or their proteolytic digestion by contaminating host proteases.

Also, as has been shown, not all the recombinant proteins used were full-length but despite this, useful MPI were obtained. This implies that MPI can be generated from gene products, which are not full length, as is frequently in cDNA expression libraries. The criteria determined should also not affect the generation of MPI from most recombinant systems, as genes cloned in either random-primed or oligo-dT-primed cDNA libraries should contain proteins, which on digestion, give peaks in this range.

In conclusion, the generation of MPI-database may have broad applications in the improved identification of proteins from many sources, such as from 2D gels, recombinant proteins, interacting proteins and whole protein complexes.

### References

Anderson L, Seilhamer J. (1997), *Electrophoresis* 18:533-537.
Ausubel et al., (1989), Current Protocols in Molecular Biology, Green Publishing Associates and Wiley Interscience, N.Y.
Büssow, K.; Cahill, D.J.; Nietfeld, W.; Bancroft, D.; Scherzinger, E.; Lehrach, H.; Walter; G. (1998) *Nucl. Acids. Res., 26,* 5007-5008.
Cahill et al. (2000), "Proteomes: From Protein Sequence to Function" in "Bridging Genomics to Proteomics", 1-17, Bios Publishing Com.
Cahill (2000), Proteomics: A Trends Guide, 47-51.
Eickhoff et al. (2000), Genome Research 10: 1230-1240.
Gobom et al. (2001), Anal. Chem. 73: 434-438.
Harlow und Lane (1988), "Antibodies, A Laboratory Manual", CSH Press, Cold Spring Harbor, USA,.
Herwig, R., Poustka, A., Müller, C., Bull, C., Lehrach, H. and O'Brien, J (1999), Large-scale clustering of cDNA-Fingerprinting data. Genome Research 1093-1105.
Lueking, A.; Holz, C.; Gotthold, C.; Lehrach, H.; Cahill, D. J. (2000), *Protein Expr. Purif., 20,* 372-378.
Meier-Ewert, S., Lange, J., Gerst, H., Herwig, R., Schmitt, A., Freund, J., Elge, T., Mott, R., Hermann, B. and Lehrach, H. (1998) Nucl. Acids Res. 26: 2216-2223.
Poustka, AJ., Herwig, R., Krause, A., Hennig, S., Meier-Ewert, S. and Lehrach, H. (1999), Genomics 59: 122-133.
Radelof, U., Hennig, S., Seranski, P., Steinfath, M., Ramser, J., Reinhardt, R., Poustka, A., Francis, F. and Lehrach, H. (1998), Nucl. Acids Res. 26: 5358-5364.
Sambrook et al. (1989), Molecular Cloning A Laboratory Manual, Cold Spring Harbor Laboratory N.Y.
Schuerenberg, S., C. Luebbert, H. Eickhoff, M. Kalkum, H. Lehrach, and E. Nordhoff (2000), *Prestructured MALDI-MS Sample Supports,* Anal. Chem. A 72 3436-3442.

## Claims

1. A method for identifying and/or characterizing a (poly)peptide comprising:
(a) analyzing a peptide map of said (poly)peptide, wherein the peptide map comprises at least 1 peptide, and its peptide primary structure fingerprint by mass spectrometry; and
(b) comparing data obtained in step (a) with a reference (poly)peptide database, said database comprising mass spectrometric data of peptide maps, wherein a peptide map comprises at least 1 peptide, and peptide primary structure fingerprint data of the peptide(s) of the peptide map, of a (poly)peptide or of a variety of (poly)peptides.

2. The method of claim 1, wherein the data obtained in step (a) are recorded as lists of digit numbers corresponding to measured molecular or fragment ion masses or mass/charge ratios.

3. The method of claim 1 or 2, wherein said reference (poly)peptide database in step (b) is produced by the steps of:
(ba) preparing a (poly)peptide sample representative of a species, a tissue, a developmental stage, a specific age, a specific time point a cell, an organelle, a sex, a disease state, a microorganism, a tissue culture cell line, a virus, a bacteriophage, an organism, a plant, an antibody, an antibody library, a protein complex or interacting proteins;
(bb) subjecting said (poly)peptide sample to one- or two-dimensional gel electrophoresis;
(bc) excising (poly)peptides from the gel;
(bd) fragmenting said (poly)peptides;
(be) analyzing the fragments obtained in step (bd) by mass spectrometry; and
(bf) storing the data obtained in step (be) in combination with the source of the corresponding (poly)peptide in a database.

4. The method of claim 1 or 2, wherein said reference (poly)peptide database in step (b) is produced by the steps of:
(ba) preparing a (poly)peptide sample representative of a species, a tissue, a developmental stage, a specific age, a.specific time point a cell, an organelle, a sex, a disease state, a microorganism, a tissue culture cell line, a virus, a bacteriophage, an organism, a plant, an antibody, an antibody library, a protein complex or interacting proteins;
(bb) subjecting said (poly)peptide sample to one- or multi-dimensional chromatographic separation steps;
(bc) fragmentation of said separated (poly)peptides;
(bd) analyzing the fragments obtained in step (bc) by mass spectrometry; and
(be) storing the data obtained in step (bd) in combination with the source of the corresponding (poly)peptide in a database.

5. The method of claim 1 or 2, wherein said reference (poly)peptide database in step (b) is produced by the steps of:
(ba) preparing a cDNA or genomic DNA library representative of a species, a tissue, a developmental stage, a cell, an organelle, a sex, a disease state, a microorganism, a tissue culture cell line, a virus, a bacteriophage, an organism, an antibody, an antibody library, a protein complex or interacting proteins;
(bb) expressing the cDNA or genomic DNA library obtained in step (ba);
(bc) isolating (poly)peptides obtained in step (bb);
(bd) fragmenting said (poly)peptides;
(be) analyzing the fragments obtained in step (bd) by mass spectrometry; and
(bf) storing the data obtained in step (be) in combination with the source of the corresponding (poly)peptide in a database.

6. The method of any one of claims 1 to 5, wherein said reference (poly)peptide database is generated from (poly)peptides isolated from their natural context.

7. The method of any one of claims 1 to 6, wherein said (poly)peptide to be identified and/or **characterized** is a recombinantly produced (poly)peptide.

8. The method of claim 7, wherein said recombinantly produced (poly)peptide is comprised in a (poly)peptide library, said library being prepared by expressing a library of nucleic acid molecules comprising a nucleic acid molecule encoding said (poly)peptide.

9. The method of any one of claims 1 to 8, wherein said (poly)peptide to be identified and/or **characterized** is part of a protein complex.

10. The method of any one of claims 1 to 9, wherein said (poly)peptide to be identified and/or **characterized** interacts with another (poly)peptide.

11. The method of any one of claims 1 to 10, wherein said (poly)peptide to be identified and/or **characterized** is present in a lysate.

12. The method of any one of claims 1 to 11, wherein said mass spectrometric method is MALDI-MS, MALDI-MS/MS, electron spray ionization (ESI), Q-TOF or post-source decay (PSD).

13. The method of any one of claims 8 to 12, wherein said library of nucleic acid molecules encode the (poly)peptides as fusion proteins.

14. The method of claim 13, wherein said fusion proteins comprise atag.

15. The method of claim 14, wherein said tag is a His-tag.

16. The method of any one of claims 8 to 15, wherein expression is inducible.

17. The method of any one of claims 8 to 16, wherein said nucleic acid molecule is cDNA.

18. The method of any one of claims 1 to 17, wherein said analysis in step (a) is, in addition to or alternatively to mass spectrometry, effected by surface plasmon resonance.

19. The method of claim 18, wherein said surface plasmon resonance is BlAcore or SELDI.

20. The method of any one of claims 8 to 19, wherein prior to expression of said library of nucleic acid molecules, the following steps are carried out:
(aa) amplifying said nucleic acid molecules;
(ab) regularly arraying said amplified nucleic acid molecules; and, optionally (ac) hybridizing the regularly arrayed nucleic acid molecules to a variety of oligonucleotides;
(ad) identifying nucleic acid molecules that hybridize to the same set of oligonucleotides; and
(ae) regularly re-arraying per set of oligonucleotides one species of nucleic acid molecules.

21. The method of claim 20, wherein the amplification in step (aa) is effected by PCR.

22. The method of any one of claims 8 to 19, wherein, after expression of said library of nucleic acid molecules, the following steps are carried out in connection with step (b):
(bi) identifying (poly)peptides which, on the basis of the comparative analysis, have a unique minimal protein identifier; and
(bii) re-arranging the clones expressing (poly)peptides identified in step (bi) regularly into an essentially non-redundant set.

23. The method of any one of claims 20 to 22, wherein said.regularly arraying and/or said regularly re-arraying is effected on a solid support.

24. The method of claim 23, wherein said solid support is a chip, a glass substrate, a filter, a membrane, a magnetic bead, a silica wafer, metal, a mass spectrometry target or a matrix.

25. The method of any one of claims 20 to 24, wherein said regularly arraying and/or said regularly re-arraying is effected on a porous surface.

26. The method of any one of claims 20 to 24, wherein said regularly arraying and/or said regularly re-arraying is effected on a non-porous surface.

27. The method of any one of claims 20 to 26, wherein said arraying and/or re-arraying is effected by an automated device.

28. The method of any one of claims 20 to 27, wherein said variety of oligonucleotides comprises at least 2 different oligonucleotides.

29. The method of any one of claims 20 to 28, wherein prior to step (aa), the following steps are carried out:
(aa') reverse transcribing mRNA from a species, a tissue, a developmental stage, a cell, an organelle, a sex, a disease state, a microorganism, a tissue culture cell line, a virus, a bacteriophage, an organism, or a plant into cDNA;
(aa") cloning cDNA, obtained in step (aa'), or genomic DNA into an expression vector.

30. The method of any one of claims 14 to 29, wherein the following further steps are carried out:
(ai) after expression of said (poly)peptide, isolating the expressed fusion proteins by means of the tag;
(aii) fragmenting the fusion proteins;
(aiii) analyzing the fragments obtained in step (aii) by mass spectrometry; and
(aiv) storing the data obtained in step (aiii) in a database.

31. The method of claim 30, wherein said isolation is effected by metal chelate affinity purification.

32. The method of claim 31, wherein said metal chelate affinity purification employs Ni-NTA ligands immobilized onto magnetic particles.

33. The method of any one of claims 20 to 32 further comprising:
(af) hybridizing genomic DNA, cDNA, PNA or RNA molecules to the optionally re-arrayed nucleic acid molecules of step (ae); and
(ag) identifying genomic DNA, cDNA, PNA or RNA molecules that hybridize to the optionally re-arrayed nucleic acid molecules on the array.

34. The method of any one of claims 8 to 33, wherein expression is effected in procaryotes.

35. The method of claim 34, wherein said procaryotes are bacteria.

36. The method of claim 35, wherein said bacteria are E. coli.

37. The method of any one of claims 8 to 33, wherein expression is effected in non-human eukaryotes or eukaryotic cells.

38. The method of claim 37, wherein said non-human eukaryotes are yeast.

39. The method of claim 38, wherein said yeast belong to the species Pichia pastoris.

40. The method of claim 37, wherein said eukaryotic cells are mammalian or insect cells.

41. The method of any one of claims 1 to 40, wherein said peptides have a molecular weight in the range of 600 to 4500 Daltons.

42. The method of claim 41, wherein said peptides have a molecular weight of 600 to 2750 Daltons.

43. The method of any one of claims 1 to 42 wherein said comparing in step (b) comprises normalization for chemical or post-translational modifications.

44. The method of claim 43, wherein said chemical modification is oxidation.

## Patentansprüche

1. Verfahren zum Identifizieren und/oder Charakterisieren eines (Poly)peptids umfassend:
(a) Analysieren eines Peptidmaps des genannten (Poly)peptids, wobei der Peptidmap mindestens 1 Peptid enthält, und seine Peptid primäre Fingerprintstruktur durch Massenspektrometrie; und
(b) Vergleichen der erhaltenen Daten aus Schritt (a) mit einer Referenz-(Poly)peptid-Datenbank, besagte Datenbank enthält massenspektrometrische Daten von Peptidmaps, wobei ein Peptidmap mindestens 1 Peptid enthält und Peptid primäre Fingerprintstrukturdaten von dem/den Peptid(en) aus der Peptidmap, eines (Poly)peptids oder einer Vielzahl von (Poly)peptiden.

2. Verfahren nach Anspruch 1, wobei die erhaltenen Daten aus Schritt (a) gespeichert sind als Listen von Ziffemummem entsprechend den gemessenen Molekular- oder Fragmentionmassen oder Masse/Ladungsverhältnissen.

3. Verfahren nach Anspruch 1 oder 2, wobei genannte Referenz-(Poly)peptid-Datenbank in Schritt (b) hergestellt ist durch die Schritte:
(ba) Präparieren einer (Poly)peptid-Probe charakteristisch für eine Spezies, ein Gewebe, ein Entwicklungsstadium, ein spezifisches Alter, ein spezifischer Zeitpunkt einer Zelle, eine Organelle, ein Geschlecht, ein Krankheitsstadium, ein Mikroorganismus, eine Gewebekultur einer Zelllinie, ein Virus, ein Bakteriophage, ein Organismus, eine Pflanze, ein Antikörper, eine Antikörperbibliothek, ein Proteinkomplex oder interagierende Proteine;
(bb) Unterwerfen besagter (Poly)peptid-Probe einer ein- oder zweidimensionalen Gelelektrophorese,
(bc) Ausschneiden der (Poly)peptide aus dem Gel,
(bd) Fragmentieren besagter (Poly)peptide,
(be) Analysieren der erhaltenen Fragmente aus Schritt (bd) durch Massenspektrometerie; und
(bf) Speichern der erhaltenen Daten aus Schritt (be) in Kombination mit der Quelle der entsprechenden (Poly)peptide in einer Datenbank.

4. Verfahren nach Anspruch 1 oder 2, wobei genannte Referenz-(Poly)peptid-Datenbank in Schritt (b) hergestellt ist durch die Schritte:
(ba) Präparieren einer (Poly)peptid-Probe charakteristisch für eine Spezies, ein Gewebe, ein Entwicklungsstadium, ein spezifisches Alter, ein spezifischer Zeitpunkt einer Zelle, eine Organelle, ein Geschlecht, ein Krankheitsstadium, ein Mikroorganismus, eine Gewebekultur einer Zelllinie, ein Virus, ein Bakteriophage, ein Organismus, eine Pflanze, ein Antikörper, eine Antikörperbibliothek, ein Proteinkomplex oder interagierende Proteine;
(bb) Unterwerfen besagter (Poly)peptid-Probe einer ein- oder multidimensionalen , chromatographischen Trennung,
(bc) Fragmentieren der besagten getrennten (Poly)peptide,
(bd) Analysieren der erhaltenen Fragmente aus Schritt (bc) durch Massenspektrometerie; und
(be) Speichern der erhaltenen Daten aus Schritt (be) in Kombination mit der Quelle der entsprechenden (Poly)peptide in einer Datenbank.

5. Verfahren nach Anspruch 1 oder 2, wobei genannte Referenz-(Poly)peptid-Datenbank in Schritt (b) hergestellt ist durch die Schritte:
(ba) Präparieren einer cDNA oder genomischen DNA-Bibliothek charakteristisch für eine Spezies, ein Gewebe, ein Entwicklungsstadium, eine Zelle, eine Organelle, ein Geschlecht, ein Krankheitsstadium, ein Mikroorganismus, eine Gewebekultur einer Zelllinie, ein Virus, ein Bakteriophage, ein Organismus, eine Pflanze, ein Antikörper, eine Antikörperbibliothek, ein Proteinkomplex oder interagierende Proteine;
(bb) Exprimieren der erhaltenen cDNA oder genomischen DNA-Bibliothek aus Schritt (ba),
(bc) Isolieren der erhaltenen (Poly)peptide aus Schritt (bb),
(bd) Fragmentieren besagter (Poly)peptide,
(be) Analysieren der erhaltenen Fragmente aus Schritt (bd) durch Massenspektrometerie; und
(bf) Speichern der erhaltenen Daten aus Schritt (be) in Kombination mit der Quelle der entsprechenden (Poly)peptide in einer Datenbank.

6. Verfahren nach einem der Ansprüche 1 bis 5, wobei die genannte Referenz-(Poly)peptid-Datenbank generiert ist aus (Poly)peptide, isoliert aus ihren naturgemäßen Kontext.

7. Verfahren nach einem der Ansprüche 1 bis 6, wobei das zu identifizierende oder zu charakterisierende besagte (Poly)peptid ein rekombinant hergestelltes (Poly)peptid ist.

8. Verfahren nach Anspruch 7, wobei das besagte rekombinant hergestellte (Poly)peptid in einer (Poly)peptid-Bibliothek enthalten ist, besagte Bibliothek ist hergestellt durch Expression einer Nukleinsäurebibliothek enthaltend eine Nukleinsäure kodierend für das besagte (Poly)peptid.

9. Verfahren nach einem der Ansprüche 1 bis 8, wobei das zu identifizierende oder zu charakterisierende besagte (Poly)peptid Teil eines Proteinkomplexes ist.

10. Verfahren nach einem der Ansprüche 1 bis 9, wobei das zu identifizierende oder zu charakterisierende besagte (Poly)peptid mit anderen (Poly)peptiden interagiert.

11. Verfahren nach einem der Ansprüche 1 bis 10, wobei das zu identifizierende oder zu charakterisierende besagte (Poly)peptid in einem Lysat vorhanden ist.

12. Verfahren nach einem der Ansprüche 1 bis 11, wobei das besagte massenspektrometrische Verfahren MALDI-MS, MALDI-MS/MS, Elektrosprayionisation (ESI), Q-TOF oder post-source decay (PSD) ist.

13. Verfahren nach einem der Ansprüche 8 bis 12, wobei besagte Nukleinsäurebibliothek kodiert für (Poly)peptide als Fusionsproteine.

14. Verfahren nach Anspruch 13, wobei besagte Fusionsproteine einen tag enthalten.

15. Verfahren nach Anspruch 14, wobei der besagte tag ein His-tag ist.

16. Verfahren nach einem der Ansprüche 8 bis 15, wobei die Expression induzierbar ist.

17. Verfahren nach einem der Ansprüche 8 bis 16, wobei besagtes Nukleinsäuremolekül cDNA ist.

18. Verfahren nach einem der Ansprüche 1 bis 17, wobei besagte Analyse in Schritt (a), zusätzlich oder alternativ zur Massenspektrometrie, durch Oberflächen Plasmonresonanz ausgeführt wird.

19. Verfahren nach Anspruch 18, wobei die besagte Oberflächen Plasmonresonanz BIA core oder SELDI ist.

20. Verfahren nach einem der Ansprüche 8 bis 19, wobei vor der Expression der besagten Nukleinsäurebibliothek, die folgenden Schritte durchgeführt werden:
(aa) Anreicherung besagter Nukleinsäuremoleküle,
(ab) reguläres Anordnen besagter angereicherter Nukleinsäuremoleküle; und, gegebenenfalls (ac) Hybridisieren der regulär angeordneten Nukleinsäuremoleküle mit einer Vielzahl von Oligonukleotiden;
(ad) Identifizieren von Nukleinsäuremolekülen, die mit dem gleichen Satz von Oligonukleotiden hybridisieren; und
(ae) reguläres Neuanordnen pro Satz von Oligonukleotiden einer Gattung von Nukleinsäuremolekülen.

21. Verfahren nach Anspruch 20, wobei die Anreicherung in Schritt (aa) durch PCR ausgeführt wird.

22. Verfahren nach einem der Ansprüche 8 bis 19, wobei nach Expression der besagten Nukleinsäurebibliothek, die folgenden Schritte in Verbindung mit Schritt (b) durchgeführt werden:
(bi) Identifizieren von (Poly)peptiden, welche auf der Basis der vergleichenden Daten, einen eindeutigen minimalen Protein-Identifikator aufweisen.
(bii) Neuanordnen der Klone, die identifizierte (Poly)peptide in Schritt (bi) regulär exprimieren in einen im Wesentlichen nicht-redundanten Satz.

23. Verfahren nach einem der Ansprüche 20 bis 22, wobei das besagte reguläre Anordnen und / oder das besagte reguläre Neuanordnen auf einem festen Träger ausgeführt wird.

24. Verfahren nach Anspruch 23, wobei besagter fester Träger ein Chip, ein Glasträger, ein Filter, eine Membran, ein magnetisches Bead, ein Silica Wafer, Metall, ein massenspektrometrisches Target oder eine Matrix ist.

25. Verfahren nach einem der Ansprüche 20 bis 24, wobei das besagte reguläre Anordnen und / oder das besagte Neuanordnen auf einer porösen Oberfläche ausgeführt wird.

26. Verfahren nach einem der Ansprüche 20 bis 24, wobei das besagte reguläre Anordnen und / oder das besagte Neuanordnen auf einer nicht-porösen Oberfläche ausgeführt wird.

27. Verfahren nach einem der Ansprüche 20 bis 26, wobei das besagte reguläre Anordnen und / oder das besagte Neuanordnen durch ein automatisiertes Gerät durchgeführt wird.

28. Verfahren nach einem der Ansprüche 20 bis 27, wobei besagte Vielzahl von Oligonukleotiden mindestens zwei verschiedene Oligonukleotide umfasst.

29. Verfahren nach einem der Ansprüche 20 bis 28, wobei vor dem Schritt (aa), die folgenden Schritte durchgeführt werden:
(aa') reverses Transkribieren von mRNA aus einer Spezies, einem Gewebe, einem Entwicklungsstadium, einer Zelle, einer Organelle, einem Geschlecht, einem Krankheitsstadium, einem Mikroorganismus, einer Gewebekultur einer Zelllinie, einem Virus, einem Bakteriophage, einem Organismus oder einer Pflanze in cDNA;
(aa") Klonieren der erhaltenen cDNA aus Schritt (aa') oder genomischer DNA in einen Expressionsvektor.

30. Verfahren nach einem der Ansprüche 14 bis 29, wobei die folgenden weiteren Schritte durchgeführt werden:
(ai) nach Expression des besagten (Poly)peptids, isolieren des exprimieretn Fusionsproteins mittels des tags;
(aii) Fragmentieren des Fusionsproteins;
(aiii) Analysieren der erhaltenen Fragmente aus Schritt (aii) durch Massenspektrometrie; und
(aiv) Speichern der erhaltenen Daten aus Schritt (aiii) in einer Datenbank.

31. Verfahren nach Anspruch 30, wobei besagte Isolation durch Metall-Chelat Affinitätsreinigung durchgeführt wird.

32. Verfahren nach Anspruch 31, wobei besagte Metall-Chelat Affinitätsreinigung Ni-NTA Liganden immobilisert auf magnetische Partikel verwendet.

33. Verfahren nach einem der Ansprüche 20 bis 32 weiter umfassend:
(af) Hybridisieren genomischer DNA, cDNA, PNA oder RNA Moleküle mit gegebenenfalls neuangeordneten Nukleinsäuremolekülen aus Schritt (ae); und
(ag) Identifizieren genomischer DNA, cDNA, PNA oder RNA Moleküle, die mit den gegebenenfalls neuangeordneten Nukleinsäuremolekülen auf der Anordnung.

34. Verfahren nach einem der Ansprüche 8 bis 33, wobei die Expression in Prokaryonten ausgeführt wird.

35. Verfahren nach Anspruch 34, wobei die Prokaryonten Bakterien sind.

36. Verfahren nach Anspruch 35, wobei die besagten Bakterien E. coli sind.

37. Verfahren nach einem der Ansprüche 8 bis 33, wobei die Expression in nicht-humanen Eukaryonten oder eukaryotischen Zellen ausgeführt wird.

38. Verfahren nach Anspruch 37, wobei besagte nicht-humane Eukaryonten Hefen sind.

39. Verfahren nach Anspruch 38, wobei besagte Hefe der Spezies Pichia pastoris angehört.

40. Verfahren nach Anspruch 37, wobei besagte eukaryontische Zelle Säugetier- oder Insektenzellen sind.

41. Verfahren nach einem der Ansprüche 1 bis 40, wobei besagte Peptide ein Molekulargewicht im Bereich von 600 bis 4500 Dalton aufweisen.

42. Verfahren nach Anspruch 41, wobei besagte Peptide ein Molekulargewicht im Bereich von 600 bis 2750 Dalton aufweisen.

43. Verfahren nach einem der Ansprüche 1 bis 42, wobei besagtes Vergleichen in Schritt (b) eine Normalisierung für chemische oder post-translationale Modifikationen enthält.

44. Verfahren nach Anspruch 43, wobei besagte chemische Modifikation die Oxidation ist.

## Revendications

1. Procédé d'identification et/ou de caractérisation d'un (poly)peptide comprenant:
(a) l'analyse d'une carte peptidique dudit (poly)peptide, dans lequel la carte peptidique comprend au moins 1 peptide, et de son empreinte de structure peptidique primaire par spectrométrie de masse; et
(b) la comparaison des données obtenues dans l'étape (a) à une base de données de (poly)peptides de référence, ladite base de données comprenant des données de spectrométrie de masse de cartes peptidiques, dans lequel une carte peptidique comprend au moins 1 peptide, et des données des empreintes de la structure peptidique primaire du (des) peptide(s) de la carte peptidique, d'un (poly)peptide ou d'une variété de (poly)peptides.

2. Procédé selon la revendication 1, dans lequel les données obtenues dans l'étape (a) sont enregistrées comme des listes de nombres correspondant aux masses moléculaires ou des ions fragments mesurées ou aux rapports masse/charge.

3. Procédé selon la revendication 1 ou 2, dans lequel ladite base de données de (poly)peptides de référence dans l'étape (b) est produite par les étapes consistant à:
(ba) préparer un échantillon de (poly)peptides représentatif d'une espèce, d'un tissu, d'un stade développemental, d'un âge spécifique, d'un point temporel spécifique, d'une cellule, d'une organelle, d'un sexe, d'un état pathologique, d'un microorganisme, d'une lignée cellulaire de culture tissulaire, d'un virus, d'un bactériophage, d'un organisme, d'une plante, d'un anticorps, d'une banque d'anticorps, d'un complexe protéique ou de protéines d'interaction;
(bb) soumettre ledit échantillon de (poly)peptides à une électrophorèse sur gel mono- ou bidimensionnelle;
(bc) exciser les (poly)peptides du gel;
(bd) fragmenter lesdits (poly)peptides;
(be) analyser les fragments obtenus dans l'étape (bd) par spectrométrie de masse; et
(bf) stocker les données obtenues dans l'étape (be) en combinaison avec la source du (poly)peptide correspondant dans une base de données.

4. Procédé selon la revendication 1 ou 2, dans lequel ladite base de données de (poly)peptides de référence dans l'étape (b) est produite par les étapes consistant à:
(ba) préparer un échantillon de (poly)peptides représentatif d'une espèce, d'un tissu, d'un stade développemental, d'un âge spécifique, d'un point temporel spécifique, d'une cellule, d'une organelle, d'un sexe, d'un état pathologique, d'un microorganisme, d'une lignée cellulaire de culture tissulaire, d'un virus, d'un bactériophage, d'un organisme, d'une plante, d'un anticorps, d'une banque d'anticorps, d'un complexe protéique ou de protéines d'interaction;
(bb) soumettre ledit échantillon de (poly)peptides à des étapes de séparation chromatographique mono- ou multi-dimensionnelle;
(bc) fragmenter lesdits (poly)peptides séparés;
(bd) analyser les fragments obtenus dans l'étape (bc) par spectrométrie de masse; et
(be) stocker les données obtenues dans l'étape (bd) en combinaison avec la source du (poly)peptide correspondant dans une base de données.

5. Procédé selon la revendication 1 ou 2, dans lequel ladite base de données de (poly)peptides de référence dans l'étape (b) est produite par les étapes consistant à:
(ba) préparer une banque d'ADNc ou d'ADN génomique représentative d'une espèce, d'un tissu, d'un stade développemental, d'une cellule, d'une organelle, d'un sexe, d'un état pathologique, d'un microorganisme, d'une lignée cellulaire de culture tissulaire, d'un virus, d'un bactériophage, d'un organisme, d'un anticorps, d'une banque d'anticorps, d'un complexe protéique ou de protéines d'interaction;
(bb) exprimer la banque d'ADNc ou d'ADN génomique obtenue dans l'étape (ba);
(bc) isoler les (poly)peptides obtenus dans l'étape (bb);
(bd) fragmenter lesdits (poly)peptides;
(be) analyser les fragments obtenus dans l'étape (bd) par spectrométrie de masse; et
(bf) stocker les données obtenues dans l'étape (be) en combinaison avec la source du (poly)peptide correspondant dans une base de données.

6. Procédé selon l'une quelconque des revendications 1 à 5, dans lequel ladite base de données de (poly)peptides de référence est générée à partir de (poly)peptides isolés de leur contexte naturel.

7. Procédé selon l'une quelconque des revendications 1 à 6, dans lequel ledit (poly)peptide à identifier et/ou caractériser est un (poly)peptide produit par voie de recombinaison.

8. Procédé selon la revendication 7, dans lequel ledit (poly)peptide produit par voie de recombinaison est compris dans une banque de (poly)peptides, ladite banque étant préparée par l'expression d'une banque de molécules d'acide nucléique comprenant une molécule d'acide nucléique codant pour ledit (poly)peptide.

9. Procédé selon l'une quelconque des revendications 1 à 8, dans lequel ledit (poly)peptide à identifier et/ou caractériser est une partie d'un complexe protéique.

10. Procédé selon l'une quelconque des revendications 1 à 9, dans lequel ledit (poly)peptide à identifier et/ou caractériser interagit avec un autre (poly)peptide.

11. Procédé selon l'une quelconque des revendications 1 à 10, dans lequel ledit (poly)peptide à identifier et/ou caractériser est présent dans un lysat.

12. Procédé selon l'une quelconque des revendications 1 à 11, dans lequel ledit procédé de spectrométrie de masse est la MALDI-SM, la MALDI-SM/SM, l'ionisation par électro-vaporisation (ESI), le Q-TOF ou la décomposition après la source (PSD).

13. Procédé selon l'une quelconque des revendications 8 à 12, dans lequel ladite banque de molécules d'acide nucléique code pour les (poly)peptides sous la forme de protéines de fusion.

14. Procédé selon la revendication 13, dans lequel lesdites protéines de fusion comprennent un marqueur.

15. Procédé selon la revendication 14, dans lequel ledit marqueur est un marqueur His.

16. Procédé selon l'une quelconque des revendications 8 à 15, dans lequel l'expression est inductible.

17. Procédé selon l'une quelconque des revendications 8 à 16, dans lequel ladite molécule d'acide nucléique est de l'ADNc.

18. Procédé selon l'une quelconque des revendications 1 à 17, dans lequel ladite analyse dans l'étape (a) est, outre la ou en variante de la spectrométrie de masse, réalisée par résonance plasmonique de surface.

19. Procédé selon la revendication 18, dans lequel ladite résonance plasmonique de surface est le BIAcore ou la SELDI.

20. Procédé selon l'une quelconque des revendications 8 à 19, dans lequel avant l'expression de ladite banque de molécules d'acide nucléique, les étapes suivantes sont réalisées:
(aa) l'amplification desdites molécules d'acide nucléique;
(ab) la répartition régulière desdites molécules d'acide nucléique amplifiées ; et, facultativement (ac) l'hybridation des molécules d'acide nucléique régulièrement réparties à une variété d'oligonucléotides;
(ad) l'identification des molécules d'acide nucléique qui s'hybrident au même jeu d'oligonucléotides; et
(ae) la re-réparation régulière par jeu d'oligonucléotides d'une espèce de molécules d'acide nucléique.

21. Procédé selon la revendication 20, dans lequel l'amplification dans l'étape (aa) est réalisée par PCR.

22. Procédé selon l'une quelconque des revendications 8 à 19, dans lequel, après l'expression de ladite banque de molécules d'acide nucléique, les étapes suivantes sont réalisées en connexion avec l'étape (b):
(bi) l'identification des (poly)peptides qui, sur la base de l'analyse comparative, ont un identificateur protéique minimal unique; et
(bii) le réarrangement des clones exprimant les (poly)peptides identifiés dans l'étape (bi) régulièrement en un jeu sensiblement non redondant.

23. Procédé selon l'une quelconque des revendications 20 à 22, dans lequel ladite répartition régulière et/ou ladite re-répartition régulière sont réalisées sur un support solide.

24. Procédé selon la revendication 23, dans lequel ledit support solide est une puce, un substrat en verre, un filtre, une membrane, une bille magnétique, une galette de silice, un métal, une cible de spectrométrie de masse ou une matrice.

25. Procédé selon l'une quelconque des revendications 20 à 24, dans lequel ladite répartition régulière et/ou ladite re-répartition régulière sont réalisées sur une surface poreuse.

26. Procédé selon l'une quelconque des revendications 20 à 24, dans lequel ladite répartition régulière et/ou ladite re-répartition régulière sont réalisées sur une surface non poreuse.

27. Procédé selon l'une quelconque des revendications 20 à 26, dans lequel ladite répartition et/ou re-répartition sont réalisées par un dispositif automatique.

28. Procédé selon l'une quelconque des revendications 20 à 27, dans lequel ladite variété d'oligonucléotides comprend au moins 2 oligonucléotides différents.

29. Procédé selon l'une quelconque des revendications 20 à 28, dans lequel avant l'étape (aa), les étapes suivantes sont réalisées:
(aa')la transcription inverse facultative de l'ARNm d'une espèce, d'un tissu, d'un stade développemental, d'une cellule, d'une organelle, d'un sexe, d'un état pathologique, d'un microorganisme, d'une lignée cellulaire de culture tissulaire, d'un virus, d'un bactériophage, d'un organisme, ou d'une plante en ADNc;
(aa")le clonage de l'ADNc, obtenu dans l'étape (aa'), ou de l'ADN génomique dans un vecteur d'expression.

30. Procédé selon l'une quelconque des revendications 14 à 29, dans lequel les étapes supplémentaires suivantes sont réalisées:
(ai) après l'expression dudit (poly)peptide, l'isolement des protéines de fusion exprimées par les moyens du marqueur;
(aii)la fragmentation des protéines de fusion;
(aiii) l'analyse des fragments obtenus dans l'étape (aii) par spectrométrie de masse; et
(aiv)le stockage des données obtenues dans l'étape (aiii) dans une base de données.

31. Procédé selon la revendication 30, dans lequel ledit isolement est réalisé par purification par affinité sur un métal chélaté.

32. Procédé selon la revendication 31, dans lequel ladite purification par affinité sur un métal chélaté utilise des ligands Ni-NTA immobilisés sur des particules magnétiques.

33. Procédé selon l'une quelconque des revendications 20 à 32, comprenant en outre:
(af) l'hybridation des molécules d'ADN génomique, d'ADNc, d'APN ou d'ARN aux molécules d'acide nucléique facultativement re-réparties de l'étape (ae); et
(ag) l'identification des molécules d'ADN génomique, d'ADNc, d'APN ou d'ARN qui s'hybrident aux molécules d'acide nucléique facultativement re-réparties sur la matrice.

34. Procédé selon l'une quelconque des revendications 8 à 33, dans lequel l'expression est réalisée dans des procaryotes.

35. Procédé selon la revendication 34, dans lequel lesdits procaryotes sont des bactéries.

36. Procédé selon la revendication 35, dans lequel lesdites bactéries sont *E. coli.*

37. Procédé selon l'une quelconque des revendications 8 à 33, dans lequel l'expression est réalisée dans des eucaryotes non humains ou dans des cellules eucaryotes.

38. Procédé selon la revendication 37, dans lequel lesdits eucaryotes non humains sont la levure.

39. Procédé selon la revendication 38, dans lequel ladite levure appartient à l'espèce *Pichia pastoris.*

40. Procédé selon la revendication 37, dans lequel lesdites cellules eucaryotes sont des cellules de mammifères ou d'insectes.

41. Procédé selon l'une quelconque des revendications 1 à 40, dans lequel lesdits peptides ont une masse moléculaire dans la plage de 600 à 4500 daltons.

42. Procédé selon la revendication 41, dans lequel lesdits peptides ont une masse moléculaire de 600 à 2750 daltons.

43. Procédé selon l'une quelconque des revendications 1 à 42, dans lequel ladite comparaison dans l'étape (b) comprend la normalisation des modifications chimiques ou post-traductionnelles.

44. Procédé selon la revendication 43, dans lequel ladite modification chimique est l'oxydation.
